(19) 

**Europäisches Patentamt**
**European Patent Office**
**Office européen des brevets**

(11) **EP 2 320 929 B1**

(12) **EUROPEAN PATENT SPECIFICATION**

(45) Date of publication and mention
of the grant of the patent:
**29.05.2019 Bulletin 2019/22**

(51) Int Cl.:
*A61P 43/00* (2006.01)     *A61P 19/00* (2006.01)
*A61P 1/00* (2006.01)      *A61P 37/00* (2006.01)
*A61P 17/00* (2006.01)     *A61K 38/17* (2006.01)

(21) Application number: **09780789.5**

(22) Date of filing: **17.07.2009**

(86) International application number:
**PCT/EP2009/059251**

(87) International publication number:
**WO 2010/007165 (21.01.2010 Gazette 2010/03)**

(54) **HUMAN BETA DEFENSINS FOR USE IN THE TREATMENT OF INFLAMMATORY DISEASES**

MENSCHLICHEN BETA-DEFENSINEN ZUR BEHANDLUNG VON ENTZÜNDLICHEN
ERKRANKUNGEN

BÊTA-DÉFENSINES HUMAINES POUR SON UTILISATION DANS LE TRAITEMENT DES
MALADIES INFLAMMATOIRES

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**
Designated Extension States:
**AL BA RS**

(30) Priority: **18.07.2008   EP 08160761
15.08.2008   EP 08162486
03.09.2008   EP 08163614
15.05.2009   EP 09160448**

(43) Date of publication of application:
**18.05.2011   Bulletin 2011/20**

(73) Proprietor: **Defensin Therapeutics ApS
2200 København N (DK)**

(72) Inventors:
• **KJAER, Tanja Maria Rosenkilde
DK-2840 Holte (DK)**
• **KRUSE, Thomas
DK-2200 Copenhagen N (DK)**
• **MYGIND, Per Holse
DK-3500 Vaerloese (DK)**
• **BRINCH, Karoline Sidelmann
DK-2400 Copenhagen NV (DK)**
• **KJAERULFF, Soeren
DK-2840 Holte (DK)**
• **ANDERSEN, Birgitte
DK-2880 Bagsvaerd (DK)**

(74) Representative: **Høiberg P/S
Adelgade 12
1304 Copenhagen K (DK)**

(56) References cited:
**WO-A1-2007/007116     WO-A1-2009/033776
WO-A2-2007/081486     US-A1- 2008 194 481**

• **PAZGIER M ET AL: "Human beta-defensins"
CMLS CELLULAR AND MOLECULAR LIFE
SCIENCES, BIRKHAUSER VERLAG,
HEIDELBERG, DE, vol. 63, no. 11, 1 June 2006
(2006-06-01), pages 1294-1313, XP002401722
ISSN: 1420-682X**
• **Marc Feldmann ET AL: "TNF defined as a
therapeutic target for rheumatoid arthritis and
other autoimmune diseases", Nature Medicine,
vol. 9, no. 10, 1 October 2003 (2003-10-01), pages
1245-1250, XP055072981, ISSN: 1078-8956, DOI:
10.1038/nm939**
• **FINNEGAN ALISON ET AL: "Collagen-induced
arthritis is exacerbated in IL-10-deficient mice",
ARTHRITIS RESEARCH AND THERAPY, BIOMED
CENTRAL, LONDON, GB, vol. 5, no. 1, 21 October
2002 (2002-10-21), pages R18-R24, XP021020680,
ISSN: 1478-6354, DOI: 10.1186/AR601**

**(Cont. next page)**

- FINNEGAN A ET AL: "Proteoglycan (aggrecan)-induced arthritis in BALB/c mice is a Th1-type disease regulated by Th2 cytokines.", JOURNAL OF IMMUNOLOGY (BALTIMORE, MD. : 1950) 15 NOV 1999, vol. 163, no. 10, 15 November 1999 (1999-11-15), pages 5383-5390, ISSN: 0022-1767
- ZHANG XINGMIN ET AL: "IL-10 is involved in the suppression of experimental autoimmune encephalomyelitis by CD25+CD4+ regulatory T cells.", INTERNATIONAL IMMUNOLOGY, vol. 16, no. 2, February 2004 (2004-02), pages 249-256, ISSN: 0953-8178
- ASADULLAH KHUSRU ET AL: "IL-10 is a key cytokine in psoriasis. Proof of principle by IL-10 therapy: A new therapeutic approach", JOURNAL OF CLINICAL INVESTIGATION, vol. 101, no. 4, 15 February 1998 (1998-02-15), pages 783-794, ISSN: 0021-9738
- DUVALLET EMILIE ET AL: "Interleukin-23: A key cytokine in inflammatory diseases", ANNALS OF MEDICINE, vol. 43, no. 7, November 2011 (2011-11), pages 503-511, ISSN: 0785-3890(print)

EP 2 320 929 B1

SEQUENCE LISTING

<110> Novozymes A/S

<120> Treatment of inflammatory diseases with mammal beta defensins

<130> 11464.204-WO

<160> 6

<170> PatentIn version 3.5

<210> 1
<211> 36
<212> PRT
<213> Homo sapiens

<220>
<221> mat peptide
<222> (1)..(36)

<400> 1

```
Asp His Tyr Asn Cys Val Ser Ser Gly Gly Gln Cys Leu Tyr Ser Ala
1               5                   10                  15

Cys Pro Ile Phe Thr Lys Ile Gln Gly Thr Cys Tyr Arg Gly Lys Ala
            20                  25                  30

Lys Cys Cys Lys
            35
```

<210> 2
<211> 41
<212> PRT
<213> Homo sapiens

<220>
<221> mat_peptide
<222> (1)..(41)

<400> 2

```
Gly Ile Gly Asp Pro Val Thr Cys Leu Lys Ser Gly Ala Ile Cys His
1               5                   10                  15

Pro Val Phe Cys Pro Arg Arg Tyr Lys Gln Ile Gly Thr Cys Gly Leu
                20              25                  30

Pro Gly Thr Lys Cys Cys Lys Lys Pro
            35                  40
```

<210> 3
<211> 45
<212> PRT
<213> Homo sapiens

<220>
<221> mat_peptide
<222> (1)..(45)

<400> 3

```
Gly Ile Ile Asn Thr Leu Gln Lys Tyr Tyr Cys Arg Val Arg Gly Gly
1               5                   10                  15

Arg Cys Ala Val Leu Ser Cys Leu Pro Lys Glu Glu Gln Thr Gly Lys
                20              25                  30

Cys Ser Thr Arg Gly Arg Lys Cys Cys Arg Arg Lys Lys
            35                  40                  45
```

<210> 4
<211> 50
<212> PRT
<213> Homo sapiens

<220>
<221> mat_peptide
<222> (1)..(50)

<400> 4

```
        Glu Phe Glu Leu Asp Arg Ile Cys Gly Tyr Gly Thr Ala Arg Cys Arg
        1               5                   10                  15

        Lys Lys Cys Arg Ser Gln Glu Tyr Arg Ile Gly Arg Cys Pro Asn Thr
                    20                  25                  30

        Tyr Ala Cys Cys Leu Arg Lys Trp Asp Glu Ser Leu Leu Asn Arg Thr
                35                  40                  45

        Lys Pro
            50
```

<210> 5
<211> 37
<212> PRT
<213> Artificial

<220>
<223> hBD-4 variant consisting of amino acids 3-39 of SEQ ID NO:4

<220>
<221> mat_peptide
<222> (1)..(37)

<400> 5

```
        Glu Leu Asp Arg Ile Cys Gly Tyr Gly Thr Ala Arg Cys Arg Lys Lys
        1               5                   10                  15

        Cys Arg Ser Gln Glu Tyr Arg Ile Gly Arg Cys Pro Asn Thr Tyr Ala
                    20                  25                  30

        Cys Cys Leu Arg Lys
                35
```

<210> 6
<211> 40
<212> PRT
<213> Mus musculus

<220>
<221> mat_peptide
<222> (1)..(40)

<400> 6

```
Lys Ile Asn Asn Pro Val Ser Cys Leu Arg Lys Gly Gly Arg Cys Trp
1               5                   10                  15

Asn Arg Cys Ile Gly Asn Thr Arg Gln Ile Gly Ser Cys Gly Val Pro
            20                  25                  30

Phe Leu Lys Cys Cys Lys Arg Lys
            35                  40
```

**Description**

**BACKGROUND**

**Technical Field**

The present disclosure relates to suppression of tumor necrosis factor alpha (TNF-alpha or TNF-$\alpha$) activity by administration of mammal beta defensins, which has utility in the treatment of a variety of disorders, including the treatment of pathological conditions associated with inflammation.

**Background**

Human defensins

Among many other elements, key components of innate immunity are the antimicrobial peptides (AMPs) that individually show considerable selectivity, but collectively are able to rapidly kill a broad spectrum of bacteria, viruses and fungi. The biological significance of AMPs is emphasized by their ubiquitous distribution in nature and they are probably produced by all multicellular organisms. In humans the predominant AMPs are the defensins. The human defensins are small cationic peptides that can be divided into $\alpha$- and $\beta$-defensins based on the topology of their three intramolecular cysteine disulphide bonds. The $\alpha$-defensins can be further subdivided into those that were first isolated from neutrophil granules (HNP1-4) and those that are expressed by Paneth cells in the crypts of the small intestine (HD5 and HD6). The $\beta$-defensins are mainly produced by epithelial cells in a various of tissues and organs including the skin, trachea, gastrointestinal tract, urogenital system, kidneys, pancreas and mammary gland. The best characterized members of the $\beta$-defensin family are hBD1-3. However, using various bioinformatics tools almost 40 open reading frames encoding putative $\beta$-defensin homologues have been annotated in the human genome. Some of the human defensins are produced constitutively, whereas others are induced by proinflammatory cytokines or exogenous microbial products.

It has become increasingly clear that the human defensins in addition to their direct antimicrobial activity also have a wide range of immunomodulatory/alternative properties. These include the induction of various chemokines and cytokines, chemotactic and apoptotic activities, induction of prostaglandin, histamine and leukotriene release, inhibition of complement, stimulation of dendritic cell maturation through toll-like receptor signaling and stimulation of pathogen clearance by neutrophils. Furthermore, the human defensins also play a role in wound healing, proliferation of epithelial and fibroblast cells, angiogenesis and vasculogenesis.

There is increasing evidence that the human defensins play an important role in many infectious and inflammatory diseases. Overexpression of human defensins is often observed in inflamed and/or infected skin most likely because of local induction by microbial components or endogenous proinflammatory cytokines. In psoriasis hBD2 and hBD3 are overabundant and in lesional epithelium of patients with acne vulgaris or superficial folliculitis a significant upregulation of hBD2 has been observed. On the other hand, downregulation of hBD2 and hBD3 has been associated with atopic dermatitis. Ileal Crohn's disease has been associated with deficient expression of HD5 and HD6 and in Crohn's disease in the colon expression of hBD2-4 are downregulated.

Cytokines

Cytokines are small, secreted polypeptides from higher eukaryotes which are responsible for intercellular signal transduction and which affect the growth, division and functions of other cells. They are potent, pleiotropic polypeptides that, e.g. via corresponding receptors, act as local or systemic intercellular regulatory factors, and therefore play crucial roles in many biologic processes, such as immunity, inflammation, and hematopoiesis. Cytokines are produced by diverse cell types including fibroblasts, endothelial cells, epithelial cells, macrophages/monocytes, and lymphocytes.

TNF-$\alpha$ is implicated in various pathophysiological processes and can be either protective, as in host defense, or deleterious, as in autoimmunity. TNF-$\alpha$ is one of the key cytokines that triggers and sustains the inflammation response and TNF-$\alpha$ inactivation has proven to be important in downregulating the inflammatory reactions associated with autoimmune diseases. Upon an infection, TNF-$\alpha$ is secreted in high amounts by macrophages and it mediates the recruitment of neutrophils and macrophages to sites of infection by stimulating endothelial cells to produce adhesion molecules and by producing chemokines, which are chemotactic cytokines. TNF-$\alpha$ help activate leukocytes and other inflammatory cells and increase vascular permeability within injured tissues. TNF-$\alpha$ is mainly produced by macrophages, monocytes and dendritic cells, but also by a broad variety of other cell types including lymphoid cells, mast cells, endothelial cells, cardiac myocytes, adipose tissue, fibroblasts and neuronal tissue.

Current anti-inflammatory drugs block the action of TNF-$\alpha$ by binding to it and hereby prevents it from signaling the receptors for TNF-$\alpha$ on the surface of cells. This type of blocking has some serious side effects, of which some is

3

infections such as tuberculosis, sepsis and fungal infections and possible increased cancer incidence.

IL-10, also known as human cytokine synthesis inhibitory factor (CSIF), is also a key player in immune regulation as an anti-inflammatory cytokine. This cytokine is produced by several cell types including monocytes, macrophages, T cells, B cells, dendritic cells and mast cells. This cytokine has pleiotropic effects in immunoregulation and inflammation. It down-regulates the expression of pro-inflammatory cytokines, cytokines secreted by Th1/Th17 cells, MHC class II Ags, and costimulatory molecules on antigen-presenting cells. IL-10 is also secreted by a population of T cells called regulatory T cells (Tregs). These cells do not prevent initial T cell activation; rather, they inhibit a sustained response and prevent chronic and potentially damaging responses. In the periphery some T cells are induced to become Tregs by antigen and either IL-10 or TGF-$\beta$. Tregs induced by IL-10 are CD4+/CD25+/Foxp3- and are referred to as Tr1 cells. These cells suppress immune responses by secretion of IL-10. Recent studies have revealed a greater diversification of the T cell effector repertoire than the Th1/Th2/Treg with the identification of Th17 cells. This subpopulation has been shown to be pathogenic in several autoimmune diseases, such as Crohn's disease, ulcerative colitis, psoriasis and multiple scelerosis, previously attributed to the Th1 lineage. The cytokines secreted by Th17 are also downregulated by IL-10 and blocking of TNF prevents psoriasis by inactivating Th17 cells. The overall activity of IL-10 is anti-inflammatory and it has been shown to prevent inflammation and injury in several animal studies, however clinical IL-10 treatment remains insufficient because of difficulties in the route of IL-10 administration and its biological half-life.

Using Human Defensins to treat Inflammation

Interestingly, Crohn's disease in the small intestine has been associated with decreased levels of the paneth cell $\alpha$-defensins HD5 and HD6, whereas Crohn's disease in the colon has been associated with reduced production of the $\beta$-defensins hBD2 and hBD3 (Gersemann et al., 2008; Wehkamp et al, 2005). Furthermore, involvement of the enteric microbiota in the pathogenesis of Crohn's has been convincingly demonstrated (Swidsinski et al., 2002). Using fluorescence in situ hybridization, these researchers showed that in active Crohn's disease a drastic increase of mucosa-associated and invasive bacteria was observed, whereas these bacteria are absent from the normal small and large bowel epithelium. Together these observations have merged into a hypothesis, which suggest that in healthy persons a proper level of defensins along the intestinal epithelial barrier acts to control the composition and number of luminal bacteria and keep them away from adhering to and invading the mucosa to trigger an inflammation (Wang et al., 2007). On the other hand, in persons with an insufficient ability to produce a protective level of secreted defensins, the balance is shifted between the antimicrobial defence and the luminal bacteria. As a result, this allows a bacterial invasion into underlying intestinal tissues that induce an inflammatory state, which in turn, may develop into Crohn's disease.

Based on this hypothesis, WO 2007/081486 discloses the use of several human defensins in the treatment of inflammatory bowel disease. The inventors suggested that defensins administered orally to Crohn's patients, in a formulation that allow their release at proper locations in the intestinal lumen, would reduce the number of invading bacteria, reestablish a normal epithelial barrier function and, thus, reduce the severity of the inflammatory disease.

According to WO 2007/081486, the function of the defensins is to directly target and kill bacteria in the lumen to prevent them from invading the epithelial tissue. That is, the function of the defensins is purely as an anti-infective compound. In relation to WO/2007/081486, it is surprising that hBD2 administered parentally is able to reduce the severity of DSS induced colitis in mice, because by using this route of administration the peptide never encounters luminal bacteria. Additionally, we show here that the effect of hBD2 is a reduction of the level of the pro-inflammatory cytokines TNF$\alpha$, IL-1$\beta$ and IL-23 secreted by PBMCs. These cytokines are known to be key players in many inflammatory diseases including inflammatory bowel disease. It has been known for more than a decade that the defensins beside their antimicrobial functions also posses a range of immunomodulatory functions. However, the large majority of work on the immune modulating properties of the human defensins describes them as having primarily pro-inflammatory or immune enhancing functions (See for example, Niyonsaba et al., 2007; Bowdish et al., 2006; Lehrer, 2004). WO 2007/007116 discloses a defensin polypeptide, INSP139 which may be used to treat at long list of diseases including atherosclerosis, rheumatoid arthritis and asthma. However, the sequence similarities between this polypeptide and that of the defensins of the present disclosure are in the range of 22-36%. Hence, INSP139 belongs to a completely different class of defensins than those of the present disclosure. In addition, WO 2007/007116 provides no experimental guidance as how to prepare and administer the INSP139 polypeptide to achieve a reduction of the pro-inflammatory cytokines TNF$\alpha$, IL-1 and IL-23 and increase in anti-inflammatory cytokine IL-10.

Hence, it is truly unexpected that hBD2 administered parentally is able to reduce disease severity in inflammatory bowel disease. First of all, when administered parentally, hBD2 would never reach the intestinal lumen to encounter harmful bacteria involved in inducing the disease. Moreover, based on the large majority of published literature, one would expect that a defensin entering the blood stream would induce a pro-inflammatory rather than an anti-inflammatory response, as observed in the work presented here.

**DETAILED DESCRIPTION**

The invention is defined by the appended claims. Any embodiment not falling under the scope of the appended claims does not form part of the invention.

As an aid to understanding the preferred embodiments of the present disclosure, certain definitions are provided herein.

The term "TNF-alpha activity" as used herein is a broad term, and is to be given its ordinary and customary meaning to a person of ordinary skill in the art (and is not to be limited to a special or customized meaning), and refers without limitation to an activity or effect mediated at least in part by tumor necrosis factor alpha.

The term "suppressor" as used herein is a broad term, and is to be given its ordinary and customary meaning to a person of ordinary skill in the art (and is not to be limited to a special or customized meaning), and refers without limitation to a molecule (e.g., natural or synthetic compound) that can decrease at least one activity of TNF-alpha. In other words, a "suppressor" alters activity if there is a statistically significant change in the amount of TNF-alpha measured, in TNF-alpha activity, or in TNF-alpha detected extracellularly and/or intracellularly in an assay performed with a suppressor, compared to the assay performed without the suppressor.

In general, TNF-alpha suppressors reduce the physiological function of TNF-alpha, for example by reducing secretion of TNF-alpha, and thus are useful in the treatment of diseases where TNF-alpha may be pathogenic, directly or indirectly.

The term "IL-10 activity" as used herein is a broad term, and is to be given its ordinary and customary meaning to a person of ordinary skill in the art (and is not to be limited to a special or customized meaning), and refers without limitation to an activity or effect mediated at least in part by interleukin-10.

The term "inducer" as used herein is a broad term, and is to be given its ordinary and customary meaning to a person of ordinary skill in the art (and is not to be limited to a special or customized meaning), and refers without limitation to a molecule (e.g., natural or synthetic compound) that can increase at least one activity of IL-10. In other words, an "inducer" alters activity if there is a statistically significant change in the amount of IL-10 measured, in IL-10 activity, or in IL-10 detected extracellularly and/or intracellularly in an assay performed with an inducer, compared to the assay performed without the inducer.

In general, IL-10 inducers increase the physiological function of IL-10, and thus are useful in the treatment of diseases, which are influenced by IL-10.

The term "modification" means herein any chemical modification of human beta defensin 2. The modification(s) can be substitution(s), deletion(s) and/or insertions(s) of the amino acid(s) as well as replacement(s) of amino acid side chain(s); or use of unnatural amino acids with similar characteristics in the amino acid sequence. In particular the modification(s) can be amidations, such as amidation of the C-terminus.

The term "defensin" as used herein refers to polypeptides recognized by a person skilled in the art as belonging to the defensin class of antimicrobial peptides. To determine if a polypeptide is a defensin according to the disclosure, the amino acid sequence may be compared with the hidden markov model profiles (HMM profiles) of the PFAM database by using the freely available HMMER software package.

The PFAM defensin families include for example Defensin_1 or "Mammalian defensin" (accession no. PF00323), and Defensin_2 or Defensin_beta or "Beta Defensin" (accession no. PF00711).

The defensins of the disclosure belong to the beta defensin class. The defensins from the beta defensin class share common structural features, such as the cysteine pattern.

Examples of defensins, according to the disclosure, include human beta defensin 1 (hBD1; see SEQ ID NO:1), human beta defensin 2 (hBD2; see SEQ ID NO:2), human beta defensin 3 (hBD3; see SEQ ID NO:3), human beta defensin 4 (hBD4; see SEQ ID NO:4), and mouse beta defensin 3 (mBD3; see SEQ ID NO:6).

The relatedness between two amino acid sequences or between two nucleotide sequences is described by the parameter "identity".

For purposes of the present disclosure, the degree of identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends in Genetics 16: 276-277; http://emboss.org), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present disclosure, the degree of identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, supra; http://emboss.org), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$\text{(Identical Deoxyribonucleotides x 100)/(Length of Alignment – Total Number of Gaps in Alignment)}.$$

The term "isolated variant" or "isolated polypeptide" as used herein refers to a variant or a polypeptide that is isolated from a source. In one aspect of the present disclosure, the variant or polypeptide is at least 1% pure, preferably at least 5% pure, more preferably at least 10% pure, more preferably at least 20% pure, more preferably at least 40% pure, more preferably at least 60% pure, even more preferably at least 80% pure, and most preferably at least 90% pure, as determined by SDS-PAGE.

The term "substantially pure polypeptide" denotes herein a polypeptide preparation that contains at most 10%, preferably at most 8%, more preferably at most 6%, more preferably at most 5%, more preferably at most 4%, more preferably at most 3%, even more preferably at most 2%, most preferably at most 1%, and even most preferably at most 0.5% by weight of other polypeptide material with which it is natively or recombinantly associated. It is, therefore, preferred that the substantially pure polypeptide is at least 92% pure, preferably at least 94% pure, more preferably at least 95% pure, more preferably at least 96% pure, more preferably at least 96% pure, more preferably at least 97% pure, more preferably at least 98% pure, even more preferably at least 99%, most preferably at least 99.5% pure, and even most preferably 100% pure by weight of the total polypeptide material present in the preparation. The polypeptides of the present disclosure are preferably in a substantially pure form. This can be accomplished, for example, by preparing the polypeptide by well-known recombinant methods or by classical purification methods.

**Mammal beta defensins**

The present disclosure relates to pharmaceutical uses of mammal beta defensins, such as human beta defensins and/or mouse beta defensins, in the treatment of diseases associated with increased (pathogenic) levels of TNF-alpha, such as inflammatory diseases. The treatment is preferably associated with reduced TNF-alpha activity in the treated tissues. Thus, the mammal beta defensins of the disclosure are also referred to as TNF-alpha suppressors.

In an embodiment of the present disclosure, the mammal beta defensins of the disclosure have a degree of identity of at least 80%, preferably at least 85%, more preferably at least 90%, and most preferably at least 95% to any of the amino acid sequences of SEQ ID NO:1, SEQ ID NO:2, SEQ ID NO:3, SEQ ID NO:4, SEQ ID NO:5 and/or SEQ ID NO:6. In a preferred embodiment of the present disclosure, the mammal beta defensins of the disclosure have a degree of identity of at least 80%, preferably at least 85%, more preferably at least 90%, and most preferably at least 95% to any of the amino acid sequences of SEQ ID NO:1, SEQ ID NO:2, SEQ ID NO:3 and/or SEQ ID NO:4. In a more preferred embodiment of the present disclosure, the mammal beta defensins of the disclosure consist of human beta defensin 1 (SEQ ID NO:1), human beta defensin 2 (SEQ ID NO:2), human beta defensin 3 (SEQ ID NO:3), human beta defensin 4 (SEQ ID NO:4), a variant of human beta defensin 4 (SEQ ID NO:5) and/or mouse beta defensin 3 (SEQ ID NO:6). In an even more preferred embodiment of the present disclosure, the mammal beta defensins of the disclosure consist of human beta defensin 1 (SEQ ID NO:1), human beta defensin 2 (SEQ ID NO:2), human beta defensin 3 (SEQ ID NO:3) and/or human beta defensin 4 (SEQ ID NO:4).

In another embodiment of the present disclosure, the mammal beta defensins of the disclosure have a degree of identity of at least 80%, preferably at least 85%, more preferably at least 90%, and most preferably at least 95% to the amino acid sequence of SEQ ID NO:2. In a preferred embodiment of the present disclosure, the mammal beta defensins of the disclosure consist of human beta defensin 2 (SEQ ID NO:2).

In yet another embodiment of the present disclosure, the mammal beta defensins of the disclosure consist of human beta defensins and/or mouse beta defensins, and functionally equivalent variants thereof. Preferably, the mammal beta defensins consist of human beta defensin 1, human beta defensin 2, human beta defensin 3, human beta defensin 4 and mouse beta defensin 3, and functionally equivalent variants thereof. More preferably, the mammal beta defensins of the disclosure consist of human beta defensin 2, and functionally equivalent variants thereof.

The mammal beta defensins of the disclosure are also referred to as compounds of the preferred embodiments of the present disclosure.

In the context of the present disclosure, a "functionally equivalent variant" of a mammal (e.g. human) beta defensin is a modified mammal (e.g. human) beta defensin exhibiting approx. the same effect on TNF-alpha activity as the mammal (e.g. human) beta defensin, such as human beta defensin 2. More preferably, it also exhibits approx. the same effect on IL-10 activity as the mammal (e.g. human) beta defensin, such as human beta defensin 2.

According to the disclosure, a functionally equivalent variant of a mammal (e.g. human) beta defensin, such as human beta defensin 2, may comprise 1-5 amino acid modifications, preferably 1-4 amino acid modifications, more preferably 1-3 amino acid modifications, most preferably 1-2 amino acid modification(s), and in particular one amino acid modification, as compared to the mammal (e.g. human) beta defensin amino acid sequence, such as SEQ ID NO:2.

Preferably, amino acid modifications are of a minor nature, that is conservative amino acid substitutions or insertions that do not significantly affect the folding and/or activity of the polypeptide; single deletions; small amino- or carboxyl-terminal extensions; a small linker peptide of up to about 20-25 residues; or a small extension that facilitates purification by changing net charge or another function, such as a poly-histidine tag, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions which do not generally alter specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

In addition to the 20 standard amino acids, non-standard amino acids (such as 4-hydroxyproline, 6-*N*-methyl lysine, 2-aminoisobutyric acid, isovaline, and alpha-methyl serine) may be substituted for amino acid residues of a wild-type polypeptide. A limited number of non-conservative amino acids, amino acids that are not encoded by the genetic code, and unnatural amino acids may be substituted for amino acid residues. "Unnatural amino acids" have been modified after protein synthesis, and/or have a chemical structure in their side chain(s) different from that of the standard amino acids. Unnatural amino acids can be chemically synthesized, and preferably, are commercially available, and include pipecolic acid, thiazolidine carboxylic acid, dehydroproline, 3- and 4-methylproline, and 3,3-dimethylproline.

Essential amino acids in mammal beta defensins can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for biological activity (*i.e.,* suppresion of TNF-alpha activity) to identify amino acid residues that are critical to the activity of the molecule. See also, Hilton et al., 1996, J. Biol. Chem. 271: 4699-4708. The identities of essential amino acids can also be inferred from analysis of identities with polypeptides which are related to mammal beta defensins.

Single or multiple amino acid substitutions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochem. 30:10832-10837; U.S. Patent No. 5,223,409; WO 92/06204), and region-directed mutagenesis (Derbyshire et al., 1986, Gene 46:145; Ner et al., 1988, DNA 7:127).

An N-terminal extension of the polypeptides of the disclosure may suitably consist of from 1 to 50 amino acids, preferably 2-20 amino acids, especially 3-15 amino acids. In one embodiment of the present disclosure N-terminal peptide extension does not contain an Arg (R). In another embodiment of the present disclosure the N-terminal extension comprises a kex2 or kex2-like cleavage site as will be defined further below. In a preferred embodiment of the present disclosure the N-terminal extension is a peptide, comprising at least two Glu (E) and/or Asp (D) amino acid residues, such as an N-terminal extension comprising one of the following sequences: EAE, EE, DE and DD.

## Methods and Uses

TNF-alpha suppressors have a variety of applicable uses, as noted above. One of skill in the art will recognize that suppression has occurred when a statistically significant variation (reduction) from TNF-alpha control levels is observed.

Human beta defensin 1, human beta defensin 2, human beta defensin 3, and a variant of human beta defensin 4, were found to reduce TNF-alpha activity, and to induce IL-10 activity, in LPS- and LTA-challenged cells. Further, human beta defensin 2 was found to reduce IL-23 secretion in LPS- and LTA-challenged cells; and mouse beta defensin 3 was found to reduce TNF activity in both murine and human LPS- and LTA-challenged cells. These findings support that the tested mammal beta defensins exhibit excellent anti-inflammatory activity, in particular anti-inflammatory activity in autoimmune diseases or disorders.

Pharmaceutical compositions of the preferred embodiments of the present disclosure can be used for treating disorders which are mediated by TNF-alpha activity. Methods of treating disorders which are mediated by TNF-alpha activity, which treatment comprises administering to a subject in need of such treatment an effective amount of a mammal beta defensin, such as human beta defensin 2, e.g., in the form of a pharmaceutical composition, are also provided. Also provided are mammal beta defensins, such as human beta defensin 2, for the manufacture of a medicament, and the use of mammal beta defensins, such as human beta defensin 2, for the manufacture of a medicament, e.g., a pharmaceutical composition, for the treatment of disorders, which are mediated by TNF-alpha activity. Treatment includes treatment of an existing disease or disorder, as well as prophylaxis (prevention) of a disease or disorder.

In an embodiment of the present disclosure, the treatment results in reduced TNF-alpha activity in the treated tissues, preferably reduced TNF-alpha activity and increased IL-10 activity.

Diseases or disorders, which can be treated with compounds of the preferred embodiments of the present disclosure, e.g., by inhibition or suppression of TNF-alpha activity, include those which are mediated by TNF-alpha activity. Preferably, treatment of these disorders can benefit from reduced TNF-alpha activity and/or increased IL-10 activity. Such diseases or disorders include inflammatory diseases or disorders, allergic diseases, and autoimmune diseases. More specifically, the disorders or diseases include rheumatoid arthritis, osteoarthritis, multiple sclerosis, artherosclerosis, scleroderma (systemic sclerosis), systemic lupus erythomatodes (SLE), lupus, (acute) glomerulonephritis, asthma, such as asthma bronchiale, chronic obstructive pulmonary diseases (COPD), respiratory distress-syndrome (ARDS), inflammatory bowel disease (e.g., Crohn's Disease), colitis (e.g., ulcerative colitis), vasculitis, uveitis, dermatitis (e.g., inflammatory dermatitis), atopic dermatitis, alopecia, rhinitis (allergica), allergic conjunctivitis, myasthenia gravis, sclerodermatitis, sarcoidosis, psoriatic arthritis, ankylosing spondylitis, juvenile idiopathic arthritis, Graves disease, Sjogren's syndrome, and Behçet disease.

The TNF-alpha suppressors can be employed therapeutically in compositions formulated for administration by any conventional route, including enterally (e.g., buccal, oral, nasal, rectal), parenterally (e.g., intravenous, intracranial, intraperitoneal, subcutaneous, or intramuscular), or topically (e.g., epicutaneous, intranasal, or intratracheal). Within other embodiments of the present disclosure, the compositions described herein may be administered as part of a sustained release implant.

Within yet other embodiments of the present disclosure, compositions, of preferred embodiments of the present disclosure may be formulized as a lyophilizate, utilizing appropriate excipients that provide stability as a lyophilizate, and subsequent to rehydration.

Pharmaceutical compositions containing the TNF-alpha suppressors of preferred embodiments of the present disclosure can be manufactured according to conventional methods, e.g., by mixing, granulating, coating, dissolving or lyophilizing processes.

In another embodiment of the present disclosure, pharmaceutical compositions containing one or more TNF-alpha suppressors are provided. For the purposes of administration, the compounds of preferred embodiments of the present disclosure may be formulated as pharmaceutical compositions. Pharmaceutical compositions of preferred embodiments of the present disclosure comprise one or more TNF-alpha suppressors of preferred embodiments of the present disclosure and a pharmaceutically acceptable carrier and/or diluent.

The TNF-alpha suppressor is preferably employed in pharmaceutical compositions in an amount which is effective to treat a particular disorder, that is, in an amount sufficient to achieve decreased TNF-alpha levels or activity, symptoms, and/or preferably with acceptable toxicity to the patient. For such treatment, the appropriate dosage will, of course, vary depending upon, for example, the chemical nature and the pharmacokinetic data of a compound of the present disclosure used, the individual host, the mode of administration and the nature and severity of the conditions being treated. However, in general, for satisfactory results in larger mammals, for example humans, an indicated daily dosage is preferably from about 0.001 g to about 1.5 g, more preferably from about 0.01 g to 1.0 g; or from about 0.01 mg/kg body weight to about 20 mg/kg body weight, more preferably from about 0.1 mg/kg body weight to about 10 mg/kg body weight, for example, administered in divided doses up to four times a day. The compounds of preferred embodiments of the present disclosure can be administered to larger mammals, for example humans, by similar modes of administration at similar dosages than conventionally used with other mediators, e.g., low molecular weight inhibitors, of TNF-alpha activity.

In certain embodiments of the present disclosure, the pharmaceutical compositions of preferred embodiments of the present disclosure can include TNF-alpha suppressor(s) in an amount of about 0.5 mg or less to about 1500 mg or more per unit dosage form depending upon the route of administration, preferably from about 0.5, 0.6, 0.7, 0.8, or 0.9 mg to about 150, 200, 250, 300, 350, 400, 450, 500, 600, 700, 800, 900, or 1000 mg, and more preferably from about 1, 2, 3, 4, 5, 6, 7, 8, 9, 10, 15, 20, or 25 mg to about 30, 35, 40, 45, 50, 55, 60, 65, 70, 75, 80, 85, 90, 95, or 100 mg. In certain embodiments of the present disclosure, however, lower or higher dosages than those mentioned above may be preferred. Appropriate concentrations and dosages can be readily determined by one skilled in the art.

Pharmaceutically acceptable carriers and/or diluents are familiar to those skilled in the art. For compositions formulated as liquid solutions, acceptable carriers and/or diluents include saline and sterile water, and may optionally include antioxidants, buffers, bacteriostats, and other common additives. The compositions can also be formulated as pills, capsules, granules, tablets (coated or uncoated), (injectable) solutions, solid solutions, suspensions, dispersions, solid dispersions (e.g., in the form of ampoules, vials, creams, gels, pastes, inhaler powder, foams, tinctures, lipsticks, drops, sprays, or suppositories). The formulation can contain (in addition to one or more TNF- alpha suppressors and other optional active ingredients) carriers, fillers, disintegrators, flow conditioners, sugars and sweeteners, fragrances, preservatives, stabilizers, wetting agents, emulsifiers, solubilizers, salts for regulating osmotic pressure, buffers, diluents, dispersing and surface-active agents, binders, lubricants, and/or other pharmaceutical excipients as are known in the art. One skilled in this art may further formulate the TNF-alpha suppressors in an appropriate manner, and in accordance with accepted practices, such as those described in Remington 's Pharmaceutical Sciences, Gennaro, Ed., Mack Publishing Co., Easton, PA 1990.

TNF-alpha suppressors can be used alone, or in combination therapies with one, two, or more other pharmaceutical compounds or drug substances, and/or with one or more pharmaceutically acceptable excipient.

In preferred embodiments of the present disclosure, the TNF-alpha suppressor is present in combination with conventional drugs used to treat diseases or conditions wherein TNF-alpha is pathogenic or wherein TNF-alpha plays a pivotal or other role in the disease process. In particularly preferred embodiments of the present disclosure, pharmaceutical compositions are provided comprising one or more TNF-alpha suppressors, including, but not limited to compounds of the preferred embodiments of the present disclosure in combination with one or more additional pharmaceutical compounds, including, but not limited to drugs for the treatment of asthma or other respiratory diseases, diabetes, arthritis or other inflammatory diseases, immune disorders, or other diseases or disorders wherein TNF-alpha is pathogenic.

The TNF-alpha suppressors of preferred embodiments of the present disclosure can be used for pharmaceutical treatment alone or in combination with one or more other pharmaceutically active agents, e.g., such as agents useful in treating inflammation, or associated diseases. Such other pharmaceutically active agents include, e.g., steroids, glucocorticoids, inhibitors of other inflammatory cytokines (e.g., anti-TNF-alpha antibodies, anti-IL-1 antibodies, anti-IFN-gamma antibodies), and other cytokines such as IL-1 RA or IL-10, and other TNF-alpha inhibitors.

Combination therapies can include fixed combinations, in which two or more pharmaceutically active agents are in the same formulation; kits, in which two or more pharmaceutically active agents in separate formulations are sold in the same package, e.g., with instructions for co-administration; and free combinations in which the pharmaceutically active agents are packaged separately, but instruction for simultaneous or sequential administration are provided. Other kit components can include diagnostics, assays, multiple dosage forms for sequential or simultaneous administration, instructions and materials for reconstituting a lyophilized or concentrated form of the pharmaceutical composition, apparatus for administering the pharmaceutically active agents, and the like. For example, a pharmaceutical package is provided comprising a first drug substance which is a compound of the preferred embodiments of the present disclosure and at least one second drug substance, along with instructions for combined administration. A pharmaceutical package is also provided comprising a compound of the preferred embodiments of the present disclosure along with instructions for combined administration with at least one second drug substance. Also provided is a pharmaceutical package comprising at least one second drug substance along with instructions for combined administration with a compound of the present disclosure.

Treatment with combinations according to the preferred embodiments of the present disclosure may provide improvements or superior outcome compared with treatments by either component of the combination alone. For example, a pharmaceutical combination comprising an amount of a compound of the preferred embodiments of the present disclosure and an amount of a second drug substance can be employed, wherein the amounts are appropriate to produce a synergistic therapeutic effect. A method for improving the therapeutic utility of a compound of the preferred embodiments of the present disclosure is also provided, comprising co-administering, e.g., concomitantly or in sequence, a therapeutically effective amount of a compound of the preferred embodiments of the present disclosure and a second drug substance. A method for improving the therapeutic utility of a second drug substance is also provided comprising coadministering, e.g., concomitantly or in sequence, a therapeutically effective amount of a compound of the preferred embodiments of the present disclosure and a second drug substance. A combination of the present disclosure and a second drug substance as a combination partner can be administered by any conventional route, for example as set out above for a compound of the preferred embodiments of the present disclosure. A second drug can be administered in dosages as appropriate, e.g., in dosage ranges which are similar to those used for single treatment, or, e.g., in case of synergy, even below conventional dosage ranges.

Suitable second drug substances include chemotherapeutic drugs, especially any chemotherapeutic agent other than the TNF-alpha suppressors of preferred embodiments of the present disclosure. Such second drug substances can include, e.g., anti-inflammatory and/or immunomodulatory drugs, and the like.

Anti-inflammatory and/or immunomodulatory drugs which may be used in combination with compounds of the preferred embodiments of the present disclosure include e.g., mTOR inhibitors, including rapamycins, e.g. 40-O-(2-hydroxyethyl)-rapamycin, 32-deoxorapamycin, 16-O-substituted rapamycins such as 16-pent-2-ynyloxy-32-deoxorapamycin, 16-pent-2-ynyloxy-32 (S or R)-dihydro- rapamycin, 16-pent-2-ynyloxy-32(S or R)-dihydro-40-O-(2-hydroxyethyl)-rapamycin, 40- [3-hydroxy-2-(hydroxy-iEtaethyl)-2-methylpropanoate]-rapamycin (also known as CCI779), 40-erhoi-(tetrazolyl)-rapamycin (also known as ABT578), the so-called rapalogs, e.g., as disclosed in PCT International Application No. WO 98/02441, PCT International Application No. WO 01/14387, and PCT International Application No. WO 03/64383, such as AP23573, and compounds disclosed under the name TAFA-93 and biolimus (biolimus A9); calcineurin inhibitors, e.g., cyclosporin A or FK 506; ascomycins having immunosuppressive properties, e.g., ABT-281, ASM981; corticosteroids; cyclophosphamide; azathioprene; leflunomide; mizoribine; mycophenolic acid or salt; mycophenolate mofetil; 15-deoxyspergualine or an immunosuppressive homologue, analogue or derivative thereof; bcr-abl tyrosine kinase inhibitors; c-kit receptor tyrosine kinase inhibitors; PDGF receptor tyrosine kinase inhibitors, e.g., Gleevec (imatinib); p38 MAP kinase inhibitors, VEGF receptor tyrosine kinase inhibitors, PKC inhibitors, e.g., as disclosed in PCT International Application No. WO 02/38561 or PCT International Application No. WO 03/82859, e.g., the compound of Example 56 or 70; JAK3 kinase inhibitors, e.g., N-benzyl-3,4-dihydroxy-benzylidene-cyanoacetamide alpha-cyano-(3,4-dihydroxy)-]N-benzylcinnamamide (Tyrphostin AG 490), prodigiosin 25-C (PNUI 56804), [4-(4'-hydroxyphenyl)-amino-6,7-dimethoxyquinazoline] (WHI-PI 31), [4- (3'-bromo-4'-hydroxylphenyl)-amino-6,7-dirnethoxyquinazoline] (WHI-PI 54), [4-(3',5-dibromo-4'-hydroxylphenyl)-amino-6,7-dimethoxyquinazoline] WHI-P97, KRX-211, 3-{(3R,4R)-4-methyl-3-[methyl-(7H-pyrro]o[2,3-d]pyrimidin-4-yJ)-arnino]-pipcridin-I-yl}-3-oxo-prorhoionitrile, in free form or in a pharmaceutically acceptable salt form, e.g., mono-citrate (also called CP-690,550), or a compound as disclosed in PCT International Application No. WO 2004/052359 or PCT International Application No. WO 2005/066156; SIP receptor agonists or modulators, e.g., FTY720 optionally phosphorylated or an analog thereof, e.g., 2-amino-2-[4-(3-benzyloxyphenylthio)-2- chlorophenyl]ethyl-I,3-propanediol optionally phosphorylated or I-{4-[I-(4-cyclohexyl- 3-trifluoromethyl-benzyloxyimino)-ethyl]-2-ethyl-benzyl}-azetidine-3-carboxylic acid or its pharmaceutically acceptable salts; immunosuppressive monoclonal antibodies, e.g., monoclonal antibodies to leukocyte receptors, e.g., Blys/BAFF receptor, MHC, CD2, CD3, CD4, CD7, CD8, CD25, CD28, CD40, CD45, CD52, CD58, CD80, CD86, IL-12 receptor, IL- 17 receptor, IL-23 receptor or their ligands; other immunomodulatory compounds, e.g., a recombinant binding molecule having at least a portion of the extracellular domain of CTLA4 or a mutant thereof, e.g., an at least extracellular portion of CTLA4 or a mutant thereof joined to a non-CTLA4 protein sequence, e.g., CTLA4lg (for ex. designated ATCC 68629) or a mutant thereof, e.g., LEA29Y; adhesion molecule inhibitors, e.g., LFA-I antagonists, ICAM-I or-3 antagonists, VCAM-4 antagonists or VLA-4 antagonists, CCR9 antagonists, MIF inhibitors, 5-aminosalicylate (5-ASA) agents, such as sulfasalazine, Azulfidine®, Asacol®, Dipentum®, Pentasa©, Rowasa®, Canasa®, Colazal®, e.g., drugs containing mesalamine; e.g., mesalazine in combination with heparin; TNF-alpha inhibitors or suppressors, e.g., others than those of the present disclosure, such as antibodies which bind to TNF-alpha, e.g., infliximab (Remicade®), nitric oxide releasing non-steroidal anti-inflammatory drugs (NSAIDs), e.g., including COX-inhibiting NO- donating drugs (CINOD); phosphodiesterase, e.g., PDE4B-inhibitors, caspase inhibitors, 'multi-functional anti-inflammatory' drugs (MFAIDs), e.g., cytosolic phospholipase A2 (cPLA2) inhibitors, such as membrane-anchored phospholipase A2 inhibitors linked to glycosaminoglycans.

In other preferred embodiments of the present disclosure, one or more TNF-alpha inhibiting compounds of the preferred embodiments of the present disclosure are present in combination with one or more nonsteroidal anti-inflammatory drugs (NSAIDs) or other pharmaceutical compounds for treating arthritis or other inflammatory diseases. Preferred compounds include, but are not limited to, celecoxib; rofecoxib; NSAIDS, for example, aspirin, celecoxib, choline magnesium tri salicylate, diclofenac potassium, diclofenac sodium, diflunisal, etodolac, fenoprofen, flurbiprofen, ibuprofen, indomethacin, ketoprofen, ketorolac, melenamic acid, nabumetone, naproxen, naproxen sodium, oxaprozin, piroxicam, rofecoxib, salsalate, sulindac, and tolmetin; and corticosteroids, for example, cortisone, hydrocortisone, methylprednisolone, prednisone, prednisolone, betamethesone, beclomethasone dipropionate, budesonide, dexamethasone sodium phosphate, flunisolide, fluticasone propionate, triamcinolone acetonide, betamethasone, fluocinolone, fluocinonide, betamethasone dipropionate, betamethasone valerate, desonide, desoximetasone, fluocinolone, triamcinolone, triamcinolone acetonide, clobetasol propionate, and dexamethasone.

In particularly preferred embodiments of the present disclosure, one or more TNF-alpha inhibiting compounds are present in combination with one or more beta stimulants, inhalation corticosteroids, antihistamines, hormones, or other pharmaceutical compounds for treating asthma, acute respiratory distress, or other respiratory diseases. Preferred compounds include, but are not limited to, beta stimulants, for example, commonly prescribed bronchodilators; inhalation corticosteroids, for example, beclomethasone, fluticasone, triamcinolone, mometasone, and forms of prednisone such as prednisone, prednisolone, and methylprednisolone; antihistamines, for example, azatadine, carbinoxamine/pseudoephedrine, cetirizine, cyproheptadine, dexchlorpheniramine, fexofenadine, loratadine, promethazine, tripelennamine, brompheniramine, cholopheniramine, clemastine, diphenhydramine; and hormones, for example, epinephrine.

In particularly preferred embodiments of the present disclosure, one or more TNF-alpha inhibiting compounds are present in combination with one or more anesthetics, e.g., ethanol, bupivacaine, chloroprocaine, levobupivacaine, lidocaine, mepivacaine, procaine, ropivacaine, tetracaine, desflurane, isoflurane, ketamine, propofol, sevoflurane, codeine, fentanyl, hydromorphone, marcaine, meperidine, methadone, morphine, oxycodone, remifentanil, sufentanil, butorphanol, nalbuphine, tramadol, benzocaine, dibucaine, ethyl chloride, xylocaine, and phenazopyridine.

In particularly preferred embodiments of the present disclosure, one or more TNF-alpha inhibiting compounds are present in combination with pharmaceutical compounds for treating irritable bowel disease, such as azathioprine or corticosteroids, in a pharmaceutical composition.

In particularly preferred embodiments of the present disclosure, one or more TNF-alpha inhibiting compounds are present in combination with immunosuppresive compounds in a pharmaceutical composition. In particularly preferred embodiments of the present disclosure, one or more TNF-alpha inhibiting compounds are present in combination with one or more drugs for treating an autoimmune disorder, for example, biological response modifiers, such as, etanercept, infliximab, and other compounds that inhibit or interfere with tumor necrosis factor.

In particularly preferred embodiments of the present disclosure, one or more TNF-alpha inhibiting compounds are present in combination with steroids, including corticosteroids, for example, cortisone, hydrocortisone, methylprednisolone, prednisone, prednisolone, betamethasone, beclomethasone dipropionate, budesonide, dexamethasone sodium phosphate, flunisolide, fluticasone propionate, triamcinolone acetonide, betamethasone, fluocinolone, fluocinonide, betamethasone dipropionate, betamethasone valerate, desonide, desoximetasone, fluocinolone, triamcinolone, triamcinolone acetonide, clobetasol propionate, and dexamethasone.

In the treatment of certain diseases, it may be beneficial to treat the patient with a TNF-alpha suppressor in combination with an anesthetic, for example, ethanol, bupivacaine, chloroprocainc, levobupivacaine, lidocainc, mcpivacaine, procaine, ropivacaine, tetracaine, desflurane, isoflurane, ketamine, propofol, sevoflurane, codeine, fentanyl, hydromorphone, marcaine, meperidine, methadone, morphine, oxycodone, remifentanil, sufentanil, butorphanol, nalbuphine, tramadol, benzocaine, dibucaine, ethyl chloride, xylocaine, and phenazopyridine..

### *In vitro* synthesis

Mammal beta defensins, such as human beta defensin 2, may be prepared by *in vitro* synthesis, using conventional methods as known in the art. Various commercial synthetic apparatuses are available, for example automated synthesizers by Applied Biosystems Inc., Beckman, etc. By using synthesizers, naturally occurring amino acids may be substituted with unnatural amino acids, particularly D-isomers (or D-forms) e.g. D-alanine and D-isoleucine, diastereoisomers, side chains having different lengths or functionalities, and the like. The particular sequence and the manner of preparation will be determined by convenience, economics, purity required, and the like.

Chemical linking may be provided to various peptides or proteins comprising convenient functionalities for bonding, such as amino groups for amide or substituted amine formation, e.g. reductive amination, thiol groups for thioether or disulfide formation, carboxyl groups for amide formation, and the like.

If desired, various groups may be introduced into the peptide during synthesis or during expression, which allow for linking to other molecules or to a surface. Thus cysteines can be used to make thioethers, histidines for linking to a metal ion complex, carboxyl groups for forming amides or esters, amino groups for forming amides, and the like.

Mammal beta defensins may also be isolated and purified in accordance with conventional methods of recombinant synthesis. A lysate may be prepared of the expression host and the lysate purified using HPLC, exclusion chromatography, gel electrophoresis, affinity chromatography, or other purification technique.

### EXAMPLES

### EXAMPLE 1

### Anti-inflammatory activity of human beta defensin 2 (hBD2)

During testing of hBD2 for immunomodulatory effects it was unexpectedly observed that hBD2 had vast anti-inflammatory potential. In human PBMC cultures it was observed that treatment with hBD2 had great influence on the cytokine profile of LPS, LTA or peptidoglycan stimulated cultures. It has previously been observed that hBD2 is able to induce the proinflammatory cytokines and chemokines IL-6, IL-1β, RANTES, IP-10 and IL-8 (Niyonsaba et al. 2007, Boniotto M. et al. 2006).

Here we show that hBD2 has downregulating potential on TNF and IL-1β, two proinflammatory cytokines; and hBD2 also induces IL-10 upon induction of an inflammatory stimulus with lipopolysaccahride (LPS), lipoteichoic acid (LTA) or peptidoglycan (PGN). IL-10 is a potential anti-inflammatory cytokine and hence the resulting effect of hBD2 is anti-inflammatory. This has been observed for human PBMC, a monocytic cell line and a dendritoid cell line.

MATERIALS AND METHODS

Production of hBD2

hBD2 was produced recombinantly. A synthetic DNA fragment (DNA 2.0) encoding hBD2 was cloned into the pET-32(+) expression vector (Novagen). The resulting plasmid encoded a translational fusion peptide containing an N-terminal thioredoxin part followed by a his-tag, an enterokinase cleavage site and finally the hBD2 peptide. The expression plasmid was transformed into *E. coli* strain BL21.

An overnight culture of this strain was diluted 100 fold in TB-glycerol containing 100 μg/ml of ampicillin and grown to an OD600 of approximately 8 at 37°C and induced with 0.5 mM of IPTG for 3 hours after which the cells were harvested by centrifugation. The his-tagged trx-hBD2 fusion peptide was purified on Ni-NTA beads (QIAGEN) using standard protocols. The his-tag purified fusion peptide was subsequently dialysed over-night into enterokinase buffer (50 mM tris-HCl pH 7.5, 1 mM CaCl$_2$) and cleaved with enterokinase to release mature hBD2. The hBD2 peptide was further purified by cation-exchange chromatography using Source 15 S matrix (Amersham Biosciences). The correct molecular weight of hBD2 was verified using MALDI-TOF mass spectrometry.

Production of mBD3 (see Example 4) was carried out using an identical protocol.

The proper folding and disulphide-bridge topology of the hBD2 molecule was subsequently verified using tryptic digestion coupled with LC-MS and NMR spectroscopy.

Endotoxin was removed from the hBD2 and mBD3 preparations by preparative RP-HPLC at low pH, and the content of endotoxin was determined by a LAL assay (Endosafe KTA2) and the level was found to be below the detection limit of the assay (0.05 EU/mg). To ascertain that levels below the detection limit of the endotoxin assay were not able to stimulate PBMC, titration curves of stimulation with a very potent lipopolysaccharide (*E. coli,* O111:B4, Sigma L4391) were performed. Very low levels of this LPS (0.06 ng/ml) were able to stimulate PBMC to a detectable cytokine production.

Isolation and stimulation of PBMC.

Peripheral blood was drawn from healthy volunteers (with approval from the relevant ethical committee in Denmark). Heparinized blood was diluted 1/1 v/v with RPMI and were subjected to Ficoll density centrifugation within 2 h of drawing. Plasma was collected from the top from individual donors and was kept on ice until it was used at 2% in the culture medium (autologous culture medium). Isolated PBMC were resuspended in autologous culture medium and seeded in 96-well culture plates with 255.000 cells per well in a total of 200 μl. PBMC from the same donor were stimulated with 100, 10 or 1 μg/ml of hBD2 either alone or together with LPS at 0.6 ng/ml or 20 ng/ml (*E. coli,* O111:B4, Sigma L4391), Lipoteichoic acid (LTA) at 1.25 μg/ml (from B. subtilis, Sigma L3265) or peptidoglycan (PGN) at 40 μg/ml (from *S. aureus,* Sigma 77140). The concentrations used for stimulation were optimized on 3 different donors in initial experiments, for LPS two different concentrations were used to be sure to be on a cytokine level that is possible to modulate. In some experiments PBMC were treated with Dexamethason and Indomethacin alone and together with LPS or LTA as a control on downregulation of inflammatory cytokines. The supernatants were collected after incubation at 37°C for 24 hours, and stored at -80°C until cytokine measurement. Viability was measured by Alamar Blue (Biosource, DALL 1100) in all experiments and in some cases also by MTS (Promega) according to manufacturer's instruction and was in some experiments also judged by counting of the cells by a Nucleocounter.

Culture and stimulation of MUTZ-3

The human myeloid leukaemia-derived cell line MUTZ-3 (DSMZ, Braunschweig, Germany) was maintained in a-MEM (Sigma M4526), supplemented with 20% [volume/volume (v/v)] fetal bovine serum (Sigma F6178) and 40 ng/ml rhGM-CSF (R&D Systems 215-GM-050). These progenitor cells is in the following denoted monocyte cell line and these monocytes were stimulated with 100, 10 or 1 μg/ml of hBD2 either alone or together with LPS or LTA.

Dendritic cell differentiation

To generate a dendritoid cell line, the human myeloid leukaemia cell lines MUTZ-3 (1 x 10$^5$ cells/ml) was differentiated for 7 days in the presence of rhGM-CSF (150 ng/ml) and rhIL-4 (50 ng/ml) into immature DCs. Medium was exchanged every 2-3 days. The differentiated cell line was further stimulated with either LPS or LTA with and without hBD2 to explore the effect of hBD2 on dendritic cells.

Cytokine measurements.

Cytokine production in supernatants was measured by flow cytometry with a human inflammation cytometric bead array (CBA) according to manufacturer's instructions (BD) on a FACSarray flow cytometer. The following cytokines were measured: IL-8, IL-1β, IL-10, TNF, IL-12 p70, IL-6. In some experiments, cytokines were measured by ELISA kits from R&D systems (IL-10, TNF-α, IL-1β) according to the manufacturer' instruction.

Data analysis

All experiments were performed at least twice, with representative results shown. The data presented are expressed as mean plus/minus standard deviation (SD). Statistical significance was determined by 2-way ANOVA with the variables being treatment (hBD2, dexamethazone, etc.) and stimulation (LPS, LTA, peptidoglycan, ect.) followed by Bonferroni post-test as reported in the table legends. Differences were considered significant for $p < 0.05$.

RESULTS

The effect of hBD2 was tested on human PBMC treated with and without LPS and LTA (Tables 1, 2 and 3). Treatment with hBD2 gave a significant downregulation of TNF in stimulated cultures for all three tested concentrations (Table 1), the downregulation is dose-dependent for LPS at 0.6 ng/ml and for LTA. For IL-1$\beta$ the downregualtion was observed mostly at the highest doses (Table 2). Interestingly, IL-10 was significantly and dose-dependently upregulated (Table 3). Downregulation of proinflammatory cytokines and induction of anti-inflammatory cytokines shows a very strong anti-inflammatory potential of hBD2. Viability was measured by two different assays, in order to exclude that the anti-inflammatory effects of hBD2 is due to cytotoxic effects. In Tables 4 and 5 it can be seen that hBD2 have no cytotoxic effect on the cells, the observed effects are stimulatory effects due to stimulation with LPS or LTA that leads to proliferation of the cells. Therefore hBD2 has no cytotoxic effect on these cells.

In Tables 6, 7 and 8, supernatants from another donor were analysed for cytokines by ELISA instead of by a cytometric bead array by flowcytometry and here the same were observed, although the sensitivity of the assay is lower and the detection limit much higher and therefore the effects were not as significant.

In order to test yet another Toll-like receptor ligand, the effect of hBD2 on peptidoglycan stimulated PBMC was investigated (Tables 9 and 10). The same was observed: TNF is dose-dependently downregulated and IL-10 is dose-dependently induced.

As a positive control on downregulation of TNF, two anti-inflammatory compounds, dexamethasone and Indomethacin, were tested in the assay. The concentrations are selected so the compounds are not toxic and achievable concentration due to solubility in medium. Indomethacin only inhibited TNF (Table 11) after stimulation with LTA, whereas dexamethasone effectively downregulated TNF production, the same was observed for IL-1 (Table 13). Indomethacin is a COX-1 and COX-2 inhibitor and is a nonsteroidal anti-inflammatory drug (NSAID) used to treat mild to moderate pain and help relieve symptoms of arthritis and dexamethasone is a synthetic glucocorticoid used primarily in the treatment of inflammatory disorders and it has very potent downregualting effect on proinflammatory cytokines (Rowland et al. 1998) at very low doses, which we also observe for TNF-$\alpha$ and IL-1$\beta$. hBD2 is as effective as or better than these two anti-inflammatory compounds.

In Tables 14 and 15, the effect of hBD2 on downregulating TNF in a monocyt cell line and on dendritic cells are shown, the same is observed as was for PBMC. IL-10 was also induced for dendritic cells stimulated with hBD2 and LPS or hBD2 and LTA (results not shown).

In order to exclude that binding of hBD2 to LPS or LTA causes the downregulation of TNF and IL-1$\beta$, the effect of hBD2 on stimulation of PBMC with a syntetic ligand (Pam3CSK4 (TLR2-TLR1 ligand), InvivoGen tlrt-pms) was tested. hBD2 was able to downregulate TNF after stimulation with this ligand as well, indicating that neutralization of LPS or LTA is not responsible for the observed effect (results not shown). Moreover, stimulation of dendritic cells with a cytokine cocktail containing TNF-$\alpha$ and IL-$\alpha$ together with hBD2 had downregulating effect on IL-1$\beta$ and IL-8 and IL-6 compared to stimulation with a cytokine cocktail alone. Obviously no effect on TNF could be analyzed, due to stimulation with TNF-$\alpha$ (results not shown).

Table 1. TNF production from human peripheral blood mononuclear cells (PBMC) after treatment with LPS or LTA with and without hBD2, all samples tested on the same donor, representative experiment out of 5 donors. TNF measured by Cytometric Bead Array (CBA) on a FACSarray, *** p<0.001 compared to respective control (bold), analysed by 2-way ANOVA (N= app. 200 for each data set).

| TNF, pg/ml (SD) | Control | hBD2 100 $\mu$g/ml | hBD2 10 $\mu$g/ml | hBD2 1 $\mu$g/ml |
|---|---|---|---|---|
| Medium | **7.3** (5.9) | 2.9 (5.1) | 2.6 (6.6) | 4.2 (10.7) |
| LPS 0.6 ng/ml | **1708.6** (428.3) | 634.2 (226.1)*** | 1076.4 (278.0)*** | 944.8 (326.6)*** |
| LPS 20 ng/ml | **2572.1** (581.1) | 1733.9 (461.3)*** | 1306.6 (375.0)*** | 1526.9 (444.2)*** |
| LTA 1.25 $\mu$g/ml | **1097.4** (293.8) | 375.2 (114.2)*** | 494.7 (158.1)*** | 711.5 (282.5)*** |

Table 2. IL-1 production from human periferial blood mononuclear cells (PBMC) after treatment with LPS or LTA with and without hBD2, all samples tested on the same donor, representative experiment out of 5 donors. IL-1β measured by Cytometric bead array (CBA) on a FACSarray, *** p<0,001 analysed by 2-way ANOVA (N= app. 200 for each data set).

| IL-1β, pg/ml (SD) | Control | hBD2 100 µg/ml | hBD2 10 µg/ml | hBD2 1 µg/ml |
|---|---|---|---|---|
| Medium | **4.2** (4.7) | 5.3 (7.1) | 3.8 (5.8) | 4.1 (51.0) |
| LPS 0.6 ng/ml | **1734.3** (347.0) | 811.0 (454.4)*** | 1949.8 (396.4)*** | 1436.2 (429.7)*** |
| LPS 20 ng/ml | **2629.5** (533.7) | 1502.1 (407.5)*** | 2273.9 (486.5)*** | 1889.3 (504.8)*** |
| LTA 1.25 µg/ml | **748.5** (172.4) | 538.3 (137.3)*** | 935.3 (238.0)*** | 986.7 (738.7)*** |

Table 3. IL-10 production from human peripheral blood mononuclear cells (PBMC) after treatment with LPS or LTA with and without hBD2, all samples tested on the same donor, representative experiment out of 5 donors. IL-10 measured by Cytometric bead array (CBA) on a FACSarray, *** p<0,001, ** p<0,01, * p<0,5 analysed by 2-way ANOVA (N= app. 200 for each data set).

| IL-10, pg/ml (SD) | Control | hBD2 100 µg/ml | hBD2 10 µg/ml | hBD2 1 µg/ml |
|---|---|---|---|---|
| Medium | **2.09** (8.65) | 2.9 (4.6) | 1.6 (4.1) | 2.09 (4.3) |
| LPS 0.6 ng/ml | **63.15** (302.5) | 232.7 (61.5)*** | 325.7 (88.2)*** | 97.2 (31.1)* |
| LPS 20 ng/ml | **70.4** (22.8) | 383.3 (133.6)*** | 355.8 (99.5)*** | 111.3 (38.8)** |
| LTA 1.25 µg/ml | **14.0** (226.1) | 175.6 (57.0)*** | 136.6 (44.7)*** | 39.9 (16.9) |

Table 4. PBMC viability after 24 h of stimulation measured by a MTS assay. Values having a different subscript letter in rows are significantly different tested by 2-way ANOVA followed by Bonferroni post-test.

| Viability, MTS (Abs 490 nm (SD)) | Control | hBD2 100 µg/ml | hBD2 10 µg/ml | hBD2 1 µg/ml |
|---|---|---|---|---|
| Medium | 1.4 (0.2) | 1.2 (0.05)[a] | 1.5 (0.2)[a] | 1.3 (0.2) |
| LPS 0.6 ng/ml | 1.6 (0.02) | 1.6 (0.1)[ab] | 2.0 (0.2)[b] | 1.5 (0.2) |
| LPS 20 ng/ml | 1.5 (0.1) | 1.9 (0.2)[b] | 1.8 (0.3)[ab] | 1.6 (0.3) |

Table 5. PBMC viability measured by Alamar Blue, one representative experiment out of 5 from 5 different donors. Values having a different superscript letter in rows and values having a different superscript number in columns are significantly different tested by 2-way ANOVA followed by Bonferroni post-test.

| Viability, Alamar Blue (RFU (SD)) | Control | hBD2 100 μg/ml | hBD2 10 μg/ml | hBD2 1 μg/ml |
|---|---|---|---|---|
| Medium | 4097130 (166631) | 3950053 (34466)[a] | 3683369 (355296)[a] | 4064143 (104634) |
| LPS 0.6 ng/ml | 4279424 (336188) | 4831188 (67646)[b] | 4664362 (147776)[b] | 4230588 (139745) |
| LPS 20 ng/ml | 4604671 (125840) | 4765256 (41383)[b] | 4623818 (56643)[b] | 4561739 (138852) |
| LTA 1.25 μg/ml | 4018914 (632833)[1] | 4664185 (154023)[b,2] | 4677870 (10199)[b,2] | 4148294 (182730)[12] |

Table 6. TNF-alfa secretion from PBMC after stimulation with hBD2, LTA, LPS or combinations hereof. TNF-alfa measured by ELISA, nd: not detectable, detection limit in assay 0.01 ng/ml, * p< 0.05 compared to respective control, ** p< 0.01 compared to respective control

| TNF-$\alpha$, ng/ml (SD) | Control | hBD2 100 μg/ml | hBD2 10 μg/ml | hBD2 1 μg/ml |
|---|---|---|---|---|
| Medium | nd | nd | nd | nd |
| LPS 0.6 ng/ml | **0.99** (0.27) | 0.41 (0.03)** | 0.59 (0.08)* | 0.70 (0.18) |
| LPS 20 ng/ml | **1.44** (0.31) | 0.53 (0.01)** | 0.49 (0.05)** | 1.18 (0.42) |
| LTA 1.25 μg/ml | **0.90** (0.32) | 0.21 (0.05)* | 0.27 (0.04)* | 0.65 (0.29) |

Table 7. IL-10 secretion from PBMC after stimulation with hBD2, LTA, LPS or combinations hereof, TNF-alfa measured by ELISA, nd: not detectable, detection limit in assay 0.03 ng/ml

| IL-10, ng/ml (SD) | Control | hBD2 100 μg/ml | hBD2 10 μg/ml | hBD2 1 μg/ml |
|---|---|---|---|---|
| Medium | nd | nd | nd | nd |
| LPS 0.6 ng/ml | nd | 0.14 (0.04) | 0.04 (0.0) | nd |
| LPS 20 ng/ml | nd | 0.46 (0.04) | 0.34 (0.04) | nd |
| LTA 1.25 μg/ml | nd | nd | nd | nd |

Table 8. IL-1$\beta$ secretion from PBMC after stimulation with hBD2, LTA, LPS or combinations hereof, TNF-alfa measured by ELISA, nd: not detectable, detection limit in assay 0.016 ng/ml, ** p< 0.01 compared to respective control

| IL-1$\beta$, ng/ml (SD) | Control | hBD2 100 μg/ml | hBD2 10 μg/ml | hBD2 1 μg/ml |
|---|---|---|---|---|
| Medium | nd | nd | nd | nd |

(continued)

| IL-1β, ng/ml (SD) | Control | hBD2 100 μg/ml | hBD2 10 μg/ml | hBD2 1 μg/ml |
|---|---|---|---|---|
| LPS 0.6 ng/ml | **0.318** (0.087) | 0.275 (0.015) | 0.268 (0.039) | 0.237 (0.007) |
| LPS 20 ng/ml | **0.920** (0.267) | 0.395 (0.033)** | 0.354 (0.013)** | 0.638 (0.159) |
| LTA 1.25 μg/ml | **0.291** (0.092) | 0.281 (0.059) | 0.193 (0.019) | 0.224 (0.030) |

Table 9. TNF production from human peripheral blood mononuclear cells (PBMC) after treatment with PGN, with and without hBD2; all samples tested on the same donor. TNF measured by Cytometric Bead Array (CBA) on a FACSarray, *** $p < 0.001$ compared to respective control, analysed by 2-way ANOVA (N= app. 200 for each data set).

| TNF, pg/ml (SD) | Control | hBD2 100 μg/ml | hBD2 10 μg/ml | hBD2 1 μg/ml |
|---|---|---|---|---|
| Medium | **0.0** (4.0) | 3.6 (5.3) | 3.7 (6.2) | 3.4 (5.2) |
| PGN 40 μg/ml | **1099.1** (251.6) | 274.9 (71.6)*** | 362.0 (97.7)*** | 809.9 (246.7)*** |

Table 10. IL-10 production from human peripheral blood mononuclear cells (PBMC) after treatment with PGN, with and without hBD2; all samples tested on the same donor. TNF measured by Cytometric Bead Array (CBA) on a FACSarray, *** $p < 0.001$ compared to respective control, analysed by 2-way ANOVA (N= app. 200 for each data set).

| IL-10, pg/ml (SD) | Control | hBD2 100 μg/ml | hBD2 10 μg/ml | hBD2 1 μg/ml |
|---|---|---|---|---|
| Medium | **0.0** (4.1) | 3.0 (9.6) | 3.6 (13.1) | 3.0 (4.8) |
| PGN 40 μg/ml | **381.3** (92.3) | 1054.2 (179.3)*** | 523.4 (111.5)*** | 337.8 (89.1) |

Table 11. TNF production from human peripheral blood mononuclear cells (PBMC) after treatment with LPS or LTA, with and without hBD2 or two different controls for inhibition of TNF (Dexamethasone and Indomethacin); all samples tested on the same donor. TNF measured by Cytometric Bead Array (CBA) on a FACSarray, values underlined are significantly reduced compared to respective control (bold), analysed by 2-way ANOVA (N= app. 200 for each data set).

| TNF, ng/ml (SD) | Medium | LPS 0.6 ng/ml | LPS 20 ng/ml | LTA 1.25 μg/ml |
|---|---|---|---|---|
| Control | **0.0** (0.0) | **1.43** (0.05) | **2.84** (0.07) | **6.72** (0.14) |
| Dexamethason 35 ng/ml | 0.0 (0.0) | <u>0.038</u> (0.004) | <u>1.69</u> (0.05) | <u>1.75</u> (0.05) |
| Dexamethason 3.5 ng/ml | 0.0 (0.0) | <u>0.30</u> (0.01) | <u>0.91</u> (0.03) | <u>2.05</u> (0.06) |
| Dexamethason 0.35 ng/ml | 0.0 (0.0) | <u>0.61</u> (0.02) | 6.04 (0.14) | <u>4.73</u> (0.10) |
| Indomethacin 7.2 ug/ml | 0.0 (0.0) | 1.71 (0.07) | 2.70 (0.07) | <u>5.80</u> (0.13) |

(continued)

| TNF, ng/ml (SD) | Medium | LPS 0.6 ng/ml | LPS 20 ng/ml | LTA 1.25 μg/ml |
|---|---|---|---|---|
| Indomethacin 0.72 ug/ml | 0.0 (0.0) | 1.56 (0.04) | 7.54 (0.17) | 5.50 (0.13) |
| hBD2 1000 μg/ml | 0.0 (0.0) | 0.003 (0.002) | 0.000 (0.002) | 0.11 (0.01) |
| hBD2 100 μg/ml | 0.0 (0.0) | 0.000 (0.002) | 0.038 (0.003) | 1.15 (0.04) |
| hBD2 10 μg/ml | 0.0 (0.0) | 0.20 (0.01) | 0.35 (0.01) | 2.33 (0.06) |
| hBD2 1 μg/ml | 0.0 (0.0) | 0.17 (0.01) | 6.24 (0.14) | 3.90 (0.10) |

Table 12. IL-10 production from human peripheral blood mononuclear cells (PBMC) after treatment with LPS or LTA, with and without hBD2 or two different controls for antiinflammatory effects (Dexamethasone and Indomethacin); all samples tested on the same donor. IL-10 measured by Cytometric Bead Array (CBA) on a FACSarray, values underlined are significantly increased compared to respective control (bold), analysed by 2-way ANOVA (N= app. 200 for each data set).

| IL-10, pg/ml (SD) | Medium | LPS 0.6 ng/ml | LPS 20 ng/ml | LTA 1.25 μg/ml |
|---|---|---|---|---|
| Control | **0.0** (218.8) | **53.9** (3.1) | **123.4** (4.6) | **170.1** (5.5) |
| Dexamethason 35 ng/ml | 0.0 (1.4) | 100.4 (3.8) | 152.5 (5.2) | 175.2 (6.6) |
| Dexamethason 3.5 ng/ml | 2.7 (1.9) | 64.6 (3.3) | 122.8 (4.7) | 112.5 (3.9) |
| Dexamethason 0.35 ng/ml | 3.9 (1.9) | 46.8 (2.8) | 197.1 (7.2) | 126.6 (4.7) |
| Indomethacin 7.2 ug/ml | 0.0 (1.5) | 45.7 (2.5) | 77.9 (3.6) | 90.4 (4.9) |
| Indomethacin 0.72 ug/ml | 0.0 (1.4) | 37.3 (19.6) | 108.0 (4.4) | 84.9 (3.5) |
| hBD2 1000 μg/ml | 0.0 (1.6) | 30.8 (2.6) | 50.5 (3.2) | 465.2 (16.3) |
| hBD2 100 μg/ml | 0.0 (4.9) | 173.5 (5.7) | 885.2 (22.2) | 766.0 (21.7) |
| hBD2 10 μg/ml | 3.9 (1.7) | 165.1 (5.6) | 497.5 (13.5) | 355.8 (9.4) |
| hBD2 1 μg/ml | 0.0 (1.9) | 42.7 (2.8) | 207.0 (6.9) | 142.1 (4.9) |

Table 13. IL-1β production from human peripheral blood mononuclear cells (PBMC) after treatment with LPS or LTA, with and without hBD2 or two different controls for antiinflammatory effects (Dexamethasone and Indomethacin); all samples tested on the same donor. IL-1 measured by Cytometric Bead Array (CBA) on a FACSarray, values underlined are significantly reduced compared to respective control (bold), analysed by 2-way ANOVA (N= app. 200 for each data set).

| IL-1β, ng/ml (SD) | Medium | LPS 0.6 ng/ml | LPS 20 ng/ml | LTA 1.25 μg/ml |
|---|---|---|---|---|
| Control | **0.00** (0.06) | **3.96** (0.18) | **6.58** (0.23) | **11.47** (0.38) |
| Dexamethason 35 ng/ml | 0.00 (0.00) | 1.00 (0.03) | 2.32 (0.07) | 3.98 (0.14) |
| Dexamethason 3.5 ng/ml | 0.00 (0.00) | 1.90 (0.06) | 3.58 (0.12) | 5.22 (0.19) |
| Dexamethason 0.35 ng/ml | 0.01 (0.00) | 2.9 (0.09) | 5.56 (0.18) | 7.91 (0.28) |
| Indomethacin 7.2 ug/ml | 0.04 (0.00) | 4.1 (0.13) | 6.12 (0.23) | 8.91 (0.30) |
| Indomethacin 0.72 ug/ml | 0.01 (0.00) | 3.1 (0.18) | 6.46 (0.22) | 7.53 (0.31) |
| hBD2 1000 μg/ml | 0.01 (0.00) | 0.53 (0.02) | 1.19 (0.08) | 4.43 (0.14) |
| hBD2 100 μg/ml | 0.00 (0.00) | 0.38 (0.01) | 1.67 (0.05) | 9.12 (0.32) |
| hBD2 10 μg/ml | 0.06 (0.00) | 1.13 (0.04) | 3.58 (0.12) | 11.0 (0.37) |
| hBD2 1 μg/ml | 0.01 (0.00) | 1.83 (0.06) | 4.91 (0.19) | 8.87 (0.29) |

Table 14. TNF production in supernatant from a human monocyte cell line (MUTZ-3) after treatment with LPS or LTA, with and without hBD2. TNF measured by Cytometric Bead Array (CBA) on a FACSarray, * p< 0.05 compared to respective control, ** p< 0.01 compared to respective control, analysed by 2-way ANOVA (N= app. 200 for each data set).

| TNF, pg/ml (SD) | Control | hBD2 100 μg/ml | hBD2 10 μg/ml | hBD2 1 μg/ml |
|---|---|---|---|---|
| Medium | **0.00** (5.56) | 0.00 (5.47) | 2.60 (7.17) | 2.21 (7.88) |
| LPS 1.5 μg/ml | **6.38** (9.28) | 3.93 (6.63)* | 3.93 (6.93)* | 6.61 (9.17) |
| LTA 1.5 μg/ml | **5.28** (9.75) | 2.64 (29.19)* | 3.76 (7.72) | 1.75 (6.96)** |

Table 15. TNF production in supernatants from immature dendritic cells stimulated with LPS or LTA (to generate mature DC), with and without hBD2. TNF measured by Cytometric Bead Array (CBA) on a FACSarray, * significantly reduced p< 0.05 compared to respective control, *** significantly reduced p< 0.01 compared to respective control, analysed by 2-way ANOVA (N= app. 200 for each data set).

| TNF, pg/ml (SD) | Control | hBD2 100 µg/ml | hBD2 10 µg/ml | hBD2 1 µg/ml |
|---|---|---|---|---|
| Medium | **0.00** (1.74) | 0.00 (1.83) | 1.89 (2.15) | 4.64 (10.26) |
| LPS 1.5 µg/ml | **23.73** (3.28) | 7.66 (2.51)*** | 13.8 (2.33)*** | 18.04 (2.89)*** |
| LTA 1.5 µg/ml | **3.78** (2.26) | 5.22 (2.25) | 2.76 (2.27)* | 0.00 (1.98)*** |

## EXAMPLE 2

### Anti-inflammatory activity of hBD1, hBD2, hBD3, and a hBD4 variant

Example 2 was carried out essentially as described in Example 1. The compound rhBD2, as shown in the tables below, is recombinant hBD2, which is identical to hBD2 as used in Example 1.

The compounds hBD1, hBD2, hBD3 and hBD4 variant, as shown in the tables below, were prepared using chemical synthesis, and obtained from Peptide Institute Inc.

The amino acid sequence of recombinant hBD2 (rhBD2) is identical to the amino acid sequence of hBD2 prepared by chemical synthesis.

The hBD4 variant shown in the tables below consists of amino acids 3-39 of hBD4, and the amino acid sequence is shown as SEQ ID NO:5.

In each table, all samples were tested on the same donor. SD means standard deviation.

RESULTS

Table 16. TNF production from human peripheral blood mononuclear cells (PBMC) after treatment with LPS with and without human beta defensins, dexamethasone or Infliximab. TNF measured by Cytometric Bead Array (CBA) on a FACSarray, * p<0.05, ** p<0.01, *** p<0.001 analyzed by 2-way ANOVA and compared to non-treated cells by Bonferroni posttests.

| Test compound | Medium | | LPS 20 ng/ml | | LPS 0.6 ng/ml | |
|---|---|---|---|---|---|---|
| | TNF pg/ml (SD) | % of control | TNF pg/ml (SD) | % of control | TNF pg/ml (SD) | % of control |
| Medium (non-treated) | 1 (1) | 100% | 2164 (632) | 100% | 728 (156) | 100% |
| rhBD2 40 µg/ml | 0 (0) | - | 167 (17)*** | 8% | 74 (5)*** | 10% |
| rhBD2 10 µg/ml | 0 (0) | - | 260 (29)*** | 12% | 125 (20)** | 17% |
| rhBD2 1 µg/ml | 1 (0) | - | 918 (373)*** | 42% | 196 (104)** | 27% |
| hBD1 40 µg/ml | 0 (0) | - | 999 (116)*** | 46% | 91 (8)** | 13% |
| hBD1 10 µg/ml | 0 (1) | - | 1311 (417)*** | 61% | 203 (20)** | 28% |

(continued)

| Test compound | Medium | | LPS 20 ng/ml | | LPS 0.6 ng/ml | |
|---|---|---|---|---|---|---|
| | TNF pg/ml (SD) | % of control | TNF pg/ml (SD) | % of control | TNF pg/ml (SD) | % of control |
| hBD1 1 µg/ml | 1 (1) | - | 1395 (201)*** | 64% | 474 (187) | 65% |
| hBD2 40 µg/ml | 0 (0) | - | 52 (71)*** | 2% | 176 (103)** | 24% |
| hBD2 10 µg/ml | 0 (0) | - | 132 (179)*** | 6% | 304 (108)* | 42% |
| hBD2 1 µg/ml | 0 (0) | - | 411 (581)*** | 19% | 242 (30)* | 33% |
| HBD-3 1 µg/ml | 0 (0) | - | 451 (24)*** | 21% | 528 (98) | 73% |
| hBD4 variant 10 µg/ml | 0 (0) | - | 139 (6)*** | 6% | 211 (22)** | 29% |
| hBD4 variant 1 µg/ml | 0 (0) | - | 778 (27)*** | 36% | 468 (59) | 64% |
| Dexamethasone | 0 (0) | - | 635 (163)*** | 29% | 47 (8)*** | 6% |
| Infliximab | 0 (0) | - | 0 (0)*** | 0% | 0 (0)*** | 0% |

Table 17. IL-10 production from human peripheral blood mononuclear cells (PBMC) after treatment with LPS with and without human beta defensins, dexamethasone or Infliximab. IL-10 measured by Cytometric Bead Array (CBA) on a FACSarray, * p<0.05, ** p<0.01, *** p<0.001 analyzed by 2-way ANOVA and compared to non-treated cells by Bonferroni posttests.

| Test compound | Medium | | LPS 20 ng/ml | | LPS 0.6 ng/ml | |
|---|---|---|---|---|---|---|
| | IL-10 pg/ml (SD) | % of control | IL-10 pg/ml (SD) | % of control | IL-10 pg/ml (SD) | % of control |
| Medium (non-treated) | 0 (0) | 100% | 111 (3) | 100% | 66 (5) | 100% |
| rhBD2 40 µg/ml | 0 (0) | - | 281 (9)*** | 252% | 108 (4)* | 162% |
| rhBD2 10 µg/ml | 0 (0) | - | 243 (38)*** | 218% | 103 (14)* | 155% |
| rhBD2 1 µg/ml | 0 (0) | - | 126 (14) | 113% | 72 (9) | 108% |
| hBD1 40 µg/ml | 0 (0) | - | 113 (5) | 102% | 69 (4) | 104% |
| hBD1 10 µg/ml | 0 (0) | - | 100 (1) | 90% | 76 (13) | 114% |

(continued)

| Test compound | Medium | | LPS 20 ng/ml | | LPS 0.6 ng/ml | |
|---|---|---|---|---|---|---|
| | IL-10 pg/ml (SD) | % of control | IL-10 pg/ml (SD) | % of control | IL-10 pg/ml (SD) | % of control |
| hBD1 1 µg/ml | 0 (0) | - | 95 (17) | 85% | 71 (6) | 108% |
| hBD2 40 µg/ml | 0 (0) | - | 323 (0)*** | 290% | 131 (13)*** | 197% |
| hBD2 10 µg/ml | 0 (0) | - | 240 (0)*** | 215% | 86 (6) | 130% |
| hBD2 1 µg/ml | 0 (0) | - | 123 (0) | 110% | 53 (5) | 80% |
| hBD3 1 µg/ml | 0 (0) | - | 152 (72)* | 137% | 71 (2) | 107% |
| hBD4 variant 10 µg/ml | 0 (0) | - | 187 (9)*** | 168% | 92 (17) | 139% |
| hBD4 variant 1 µg/ml | 0 (0) | - | 175 (8)*** | 157% | 90 (14) | 136% |
| Dexamethasone | 0 (0) | - | 75 (6)* | 67% | 47 (3) | 70% |
| Infliximab | 0 (0) | - | 63 (7)** | 56% | 46 (9) | 69% |

Table 18. IL-1β production from human peripheral blood mononuclear cells (PBMC) after treatment with LPS with and without human beta defensins, dexamethasone or Infliximab. IL-1β measured by Cytometric Bead Array (CBA) on a FACSarray, *** $p < 0.001$ analyzed by 2-way ANOVA and compared to non-treated cells by Bonferroni posttests.

| Test compound | Medium | | LPS 20 ng/ml | | LPS 0.6 ng/ml | |
|---|---|---|---|---|---|---|
| | IL-1β pg/ml (SD) | % of control | IL-1β pg/ml (SD) | % of control | IL-1β pg/ml (SD) | % of control |
| Medium (non-treated) | 0 (0) | 100% | 2544 (226) | 100% | 741 (93) | 100% |
| rhBD2 40 µg/ml | 0 (0) | - | 395 (25)*** | 16% | 124 (11)*** | 17% |
| rhBD2 10 µg/ml | 0 (0) | - | 624 (37)*** | 25% | 214 (7)*** | 29% |
| rhBD2 1 µg/ml | 0 (0) | - | 1480 (154)*** | 58% | 284 (15)*** | 38% |
| hBD1 40 µg/ml | 0 (0) | - | 1599 (14)*** | 63% | 302 (3)*** | 41% |
| hBD1 10 µg/ml | 0 (0) | - | 1913 (53)*** | 75% | 401 (17)*** | 54% |
| hBD1 1 µg/ml | 0 (0) | - | 2087 (157)*** | 82% | 512 (45)** | 69% |

(continued)

| Test compound | Medium | | LPS 20 ng/ml | | LPS 0.6 ng/ml | |
|---|---|---|---|---|---|---|
| | IL-1β pg/ml (SD) | % of control | IL-1β pg/ml (SD) | % of control | IL-1β pg/ml (SD) | % of control |
| hBD2 40 μg/ml | 1 (1) | - | 316 (0)*** | 12% | 159 (2)*** | 21% |
| hBD2 10 μg/ml | 0 (0) | - | 589 (0)*** | 23% | 238 (124)*** | 32% |
| hBD2 1 μg/ml | 0 (0) | - | 1569 (0)*** | 62% | 312 (28)*** | 42% |
| hBD3 1 μg/ml | 0 (0) | - | 568 (126)*** | 22% | 331 (23)*** | 45% |
| hBD4 variant 10 μg/ml | 0 (0) | - | 463 (40)*** | 18% | 163 (5)*** | 22% |
| hBD4 variant 1 μg/ml | 0 (0) | - | 1004 (24)*** | 40% | 286 (11)*** | 39% |
| Dexamethasone | 0 (0) | - | 1120 (220)*** | 44% | 104 (8)*** | 14% |
| Infliximab | 0 (0) | - | 2704 (0) | 106% | 636 (81) | 86% |

The effects of hBD1, hBD2, hBD3 and a hBD4 variant were tested on human PBMC treated with and without LPS (Tables 16, 17 and 18). For comparison, rhBD2 was included in each setup.

TNF was downregulated for all defensins. The reduction in IL-1β secretion was comparable to TNF, although not as pronounced as TNF. Secretion of IL-10 was significantly and dose-dependently enhanced for hBD2 and the hBD4 variant.

hBD3 was also tested at 10 μg/ml and 40 μg/ml and the hBD4 variant was also tested at 40 μg/ml; however, since both molecules were toxic to the cells at the these concentrations, it was not possible to discriminate between toxic and anti-inflammatory effects.

As a positive control on downregulation of TNF, two anti-inflammatory compounds, Dexamethasone and Infliximab, were included in the setup.

CONCLUSION

All the tested human beta defensins showed anti-inflammatory potential.

**EXAMPLE 3**

**Reduction of IL-23 from human monocyte-derived dendritic cells and human PBMCs**

Example 3 was carried out essentially as described in Example 1 for human PBMCs; however, the readout was IL-23 instead of TNF, IL-1β and IL-10. Moreover, the effect of rhBD2 on human monocyte-derived dendritic cells was also investigated.

Generation of monocyte-derived dendritic cells (DCs)

The DCs were prepared according to a modified protocol originally described by Romani *et al.* Briefly, peripheral blood mononuclear cells (PBMCs) were purified from buffy coats of healthy donors by centrifugation over a Ficoll-pague (GE-healthcare) gradient. Monocytes were isolated from PBMC by positive selection of CD14+ cells by magnetic beads (Dynal, Invitrogen) according to the manufacturer's instructions. The CD14+ monocytes were cultured in 6-well plates in RPMI/2% Human AB Serum recombinant human recombinant granulocyte-macrophage colony-stimulating factor (GM-CSF, 20 ng/ml) and IL-4 (20 ng/ml)(PeproTech) for 6 days, replenishing the medium/cytokines after 2 and 5 days. After 6 days of culture the immature DCs are re-cultured into 96-well plates in a concentration of $1 \times 10^6$ cells/ml and left untreated or treated with a cocktail and/or hBD2 for a further 24 h. hBD2 was tested in four concentrations in quadruplicate. hBD2 was analyzed for its ability to suppress hDC-maturation into a proinflammatory phenotype using a proinflammatory cocktail that contained LPS (100 ng/ml) and IFN-γ (20 ng/ml). Dexamethasone was added 20 h prior to the cocktail as positive control for a compound with proven clinical anti-inflammatory activity. The incubation with hBD2 was done 4 h prior to addition of cocktail.

Cytokine ELISA

Cell culture supernatants were collected and stored at -80°C. Amounts of IL-23 was measured by standard sandwich ELISA using commercially available antibodies and standards according to the manufacturer's protocols (eBioscience).

MTT assay

A MTT based cell growth determination kit was used as a measure of cell survival after 48 h in order to evaluate if any of the cells were severely affected by treatment with vehicles, cocktail or hBD2 and was done according to the manufacturer's protocols (Sigma).

Statistical analyses

All experiments were performed at least twice, with representative results shown. The data presented are expressed as mean plus/minus standard deviation (SEM). Statistical significance was determined by 2-way ANOVA with the variables being treatment (hBD2, dexamethazone, ect.) and stimulation (LPS, LTA, peptidoglycan, ect.) followed by Bonferroni post-test as reported in the table legends. Differences were considered significant for $p < 0.05$.

RESULTS

Table 19. IL-23 (pg/ml) in supernatants of human CD14+ monocyte-derived dendritic cells stimulated with either medium (unstimulated), or LPS and IFN-γ and treated with either medium (untreated), hBD2 or Dexamehtasone, average (SEM), N=4, one representative donor out of three. * p<0.05, ** p<0.01, *** p<0.001 analyzed by 2-way ANOVA and compared to non-treated cells by Bonferroni posttests. nd: not detected (below detection limit).

| IL-23 pg/ml (SEM) | Unstimulated | LPS (100 ng/ml) and IFN-γ (20 ng/ml) |
|---|---|---|
| Untreated | 375 (96) | 3569 (130) |
| hBD2 1 μg/ml | nd | 3833 (88) |
| hBD2 10 μg/ml | 451 (121) | 3308 (169)* |
| hBD2 30 μg/ml | nd | 3042 (46)*** |
| hBD2 100 μg/ml | nd | 2145 (202)*** |
| Dexamethasone 1 μM | 424 (38) | 1147 (268)*** |

Table 20. IL-23 (pg/ml) in supernatants of human PBMC stimulated with either medium (control), 0.6 ng/ml LPS, 20 ng/ml LPS or 5 $\mu$g/ml LTA and treated hBD2, Dexamehtasone or Infliximab, average (SEM). * p<0.05, ** p<0.01, *** p<0.001 analyzed by 1-way ANOVA and compared to non-treated cells by Dunnett's Multiple Comparison posttest.

| IL-23 pg/ml (SEM) | Control | LPS 0.6 ng/ml | LPS 20 ng/ml | LTA 5 $\mu$g/ml |
|---|---|---|---|---|
| Control (non-treated) | 257 (7) | 553 (6) | 510 (5) | 762 (20) |
| hBD2 1 $\mu$g/ml | 218 (5) | 338 (10)** | 263 (5)** | 383 (20)** |
| hBD2 10 $\mu$g/ml | 211 (4) | 462 (2)* | 295 (1)** | 438 (9)** |
| hBD2 100 $\mu$g/ml | 207 (4) | 484 (7) | 488 (8) | 810 (30) |
| Dexamethasone 3.5 ng/ml | 222 (5) | 202 (5)** | 192 (1)** | 223 (1)** |
| Infliximab 1 $\mu$g/ml | 227 (10) | 356 (10)** | 373 (2)** | 349 (1)** |

As shown in Table 19, hBD2 suppresses significantly and dose-dependently IL-23 secretion from human CD14[+] monocyte-derived dendritic cells.

For human PBMC, IL-23 secretion was also significantly suppressed (Table 20). On these cells there was an inverse dose-dependency, that was found to be a bell-shaped dose-response inhibition curve when testing lower doses of hBD2 (data not shown).

This shows that hBD2 might have a suppressive effect in a cronic autoimmune condition by suppression of IL-23 secretion, as IL-23 is an important part of the inflammatory response. Th17 cells are dependent on IL-23 for their survival and expansion, and Th17 cells have been shown to be pathogenic in several autoimmune diseases, such as Crohn's disease, ulcerative colitis, psoriasis and multiple sclerosis.

## EXAMPLE 4

### Reduction of TNF secretion from PBMCs with mouse beta defensin 3 (mBD3)

Example 4 was carried out essentially as described in Example 1 for human PBMCs. Mouse beta defensin 3 (mBD3) was prepared using the same protocol as was used for production of hBD2 in Example 1. The amino acid sequence of mBD3 is shown in SEQ ID NO:6. Mouse PBMCs were prepared as described below.

Isolation and stimulation of mouse peripheral blood mononuclear cells (PBMC)

Mouse peripheral blood mononuclear cells were isolated from blood of ten NMRI mice. In short, heparinized blood was diluted 1/1 v/v with RPMI and subjected to Ficoll density centrifugation within 2 h of drawing. Plasma was collected from the top and discarded. Isolated PBMC were resuspended in culture medium (RPMI 1640 (Gibco, 42401) w/ 1% penicillin and streptomycin and 1% L-Glutamine) and seeded in 96-well culture plates with 115.500 cells per well in a total of 200 $\mu$l. PBMC from the same donor were stimulated with 100, 10 or 1 $\mu$g/ml of hBD2 or mBD3 (mouse beta defensin 3); either alone or together with 20 ng/ml LPS (*E. coli,* O111:B4, Sigma L4391). Dexamethasone was added at 3.5 ng/ml to cultures with and without LPS stimulation. The supernatants were collected after incubation at 37°C for 24 hours, and stored at -80°C until cytokine measurement.

Cytokine production in supernatants was measured by flow cytometry with a mouse inflammation cytometric bead array (CBA) according to manufacturer's instructions (BD) on a FACSarray flow cytometer.

Viability was measured by Alamar Blue (Biosource DALL 1100) after supernatant were collected.

RESULTS

Table 21. TNF production from <u>human</u> peripheral blood mononuclear cells (PBMC) after treatment with LPS with and without hBD2, all samples tested on the same donor, representative experiment out of two donors. TNF measured by Cytometric Bead Array (CBA) on a FACSarray, *** p<0.001 compared to respective control, analysed by 2-way ANOVA (N=2).

| TNF pg/ml (SEM) | Medium | LPS 20 ng/ml |
|---|---|---|
| Medium | 5 (1) | 1353 (140) |
| mBD3 1 $\mu$g/ml | 2 (0) | 384 (11)*** |
| mBD3 10 $\mu$g/ml | 2 (0) | 51 (1)*** |
| mBD3 100 $\mu$g/ml | 39 (19) | 166 (17)*** |
| hBD2 1 $\mu$g/ml | 3 (0) | 633 (110)*** |
| hBD2 10 $\mu$g/ml | 2 (0) | 359 (10)*** |
| hBD2 100 $\mu$g/ml | 2 (0) | 342 (34)*** |
| Dexamethasone 3.5 ng/ml | 1 (0) | 460 (29)*** |
| Infliximab 1 $\mu$g/ml | 0 (0) | 1 (0)*** |

Table 22. TNF production from <u>mouse</u> peripheral blood mononuclear cells (PBMC) after treatment with LPS with and without mBD3, all samples tested on the same donor, representative experiment out of two donors. TNF measured by Cytometric Bead Array (CBA) on a FACSarray, *** p<0.001 compared to respective control, analysed by 2-way ANOVA (N=2).

| TNF $\mu$g/ml (SEM) | Medium | LPS 20 ng/ml |
|---|---|---|
| Medium | 578 (3) | 2063 (77) |
| mBD3 1 $\mu$g/ml | 347 (32) | 1600 (47)*** |
| mBD3 10 $\mu$g/ml | 180 (0) | 297 (9)*** |
| mBD3 100 $\mu$g/ml | 182 (5) | 195 (6)*** |
| Dexamethasone 3.5 ng/ml | 94 (3) | 328 (8)*** |
| Infliximab 1 $\mu$g/ml | 530 (4) | 2119 (31) |

As shown in Table 21, mouse beta defensin 3 (mBD3) is downregulating the secretion of TNF from human PBMCs to the same extend as hBD2 and dexamethason. mBD3 also downregulate the secretion of TNF from mouse PBMC (Table 22).

Accordingly, in this setup, mBD3 exhibits excellent anti-inflammatory activity.

## EXAMPLE 5

### 10-Day Dextran Sodium Sulphate (DSS)-induced colitis model in the mouse

The aim of the following study was to determine the anti-inflammatory activity of human beta defensin 2 in an acute (10-days) model of inflammatory bowel disease (colitis) induced by oral dextran sodium sulphate (DSS) administration in the mouse.

The DSS colitis mouse model is a well recognized model for studying inflammatory bowel disease, as described in Kawada et al. "Insights from advances in research of chemically induced experimental models of human inflammatory bowel disease", World J. Gastroenterol., Vol. 13 (42), pp. 5581-5593 (2007); and Wirtz and Neurath "Mouse models of inflammatory bowel disease", Advanced Drug Delivery Reviews, Vol. 59 (11), 1073-1083 (2007).

MATERIALS

Test Items

Human beta defensin 2 (hBD2); see Example 1 above
Methylprednisolone 21-hemisuccinate ("prednisolone")
PBS buffer (GIBCO)

Experimental Animals

Male C57BL/6 mice (Harlan Interfauna Ibérica, Barcelona, Spain) were used in the study, as this is a species and sex that has been demonstrated to develop significant inflammation of the colon when administered a 2% solution of DSS in the drinking water over a period of 10 days.

Identification

Animals were identified by number and letter codes on their tails. Additionally, each cage was identified by a colour-coded card indicating the number and sex of the animals, the test item code or name, dose level, administration route, treatment period, group number, study code and study director's name.

Weight

The average body weight of the animals on the day of start of the study was 22.4 $\pm$ 0.16g

Acclimatization (quarantine)

Minimum of 7 days prior to the start of the study, under the same conditions as those of the main study.

Housing

On arrival, the animals were separated and housed at random in policarbonate cages (E-Type, Charles River, 255x405x197mm) with stainless steel lids.

Animals were housed in groups of five animals per cage according to their sex, in animal rooms with controlled temperature ($22\pm2$°C), lighting (12/12 hours light/darkness), air pressure, number of air renovations and relative humidity (30-70%).

The cages all had sawdust (Lignocel 3-4; Harlan Interfauna Ibérica, Spain) on the floor as litter.

Food and water

All mice had free access to a dry, pelleted standard rodent diet (Teklad Global 2014; Harlan Interfauna Ibérica, Spain).

Water was provided ad libitum in bottles. Tap water supply to the animal rooms is periodically analysed to check its composition and to detect possible contaminants (chemical and microbiological).

Equipment and Materials

Equipment:

- Animal balance Sartorius Mod. BP 2100
- Surgical dissection equipment
- Eppendorf 5415C centrifuge
- Nikon Eclipse E600FN microscope
- Hook & Tucker instruments rotamixer
- IKA UltraTurrax Homogeniser
- Sartorius Mod. BP 221S analytical balance
- ELISA microplate reader Labsystems Multiskan EX

Materials and Reagents:

- Sterile disposable syringes (1ml)
- Sterile Butterfly 25G infusion set
- Anaesthetic (Ketamine/Xylazine)
- Topical Anaesthetic cream (EMLA, Astra Zeneca)
- Dextran Sodium Sulphate 30.000-50.000 Da (MP Biomedicals)
- Phosphate Buffered Saline (PBS; Sigma)
- Neutral Buffered Formalin (VWR)
- Bovine Serum Albumin (Sigma)
- Protease Inhibitor Cocktail (Sigma)
- Mouse TNF-$\alpha$ ELISA kit (GE Healthcare)

EXPERIMENTAL PROTOCOL

Study design

Animals were divided into 5 experimental groups. Each group consisted of 10 males:

Group A: Treated with Control vehicle (PBS) i.v.
Group B: Treated with hBD2 (0.1 mg/kg i.v.)
Group C: Treated with hBD2 (1 mg/kg i.v.)
Group D: Treated with hBD2 (10 mg/kg i.v.)
Group E: Treated with methylprednisolone (1 mg/kg p.o.)

Animal allocation to all experimental groups was done in a randomized manner. A maximum of 5 mice were housed in each cage (as per Directive 86/609/EEC). All animals were weighed on their arrival at the laboratory and prior to the administration of the test items.

Administration of the Test Substance

The control vehicle and hBD2 were administered intravenously via the tail vein with the use of a sterile needle (25G) in a dosing volume of 5 ml/kg body weight as a slow bolus. The animals received one dose daily (every 24 hours) of the corresponding test item (hBD2, prednisolone or control vehicle) for 10 consecutive days.

Prednisolone was given orally at a dose of 1 mg/kg in a dosing volume of 5 ml/kg body weight, in the same dosing regime as hBD2.

EXPERIMENTAL PROCEDURE

Induction of Colitis

Colitis was induced in mice by supplementing their drinking water with DSS 2% for 7 days.

On Day 1 all mice were weighed and marked according to their experimental groups. The drinking bottle of each cage was filled with the DSS solution, making sure all bottle lids were mounted properly and that none were congested.

On Day 3 any remaining solution in the bottles was emptied and refilled with fresh DSS solution. This procedure was repeated again on Day 5.

On Day 8 any remaining solution was discarded and replaced with autoclaved water.

Animals were sacrificed 2 days later on Day 10.

Clinical Assessment (Disease Activity Index)

Daily clinical assessment of DSS-treated animals was carried out, with the calculation of a validated clinical Disease Activity Index (DAI) ranging from 0 to 4 according to the following parameters: stool consistency, presence or absence of rectal bleeding and weight loss:

| Parameter | | DAI score |
|---|---|---|
| Change in Body Weight: | <1% | 0 |
| | 1-5% | 1 |
| | 5-10% | 2 |
| | 10-15% | 3 |
| | >15% | 4 |
| Rectal Bleeding: | Negative | 0 |
| | Positive | 4 |
| Stool Consistency: | Normal | 0 |
| | Loose Stools | 2 |
| | Diarrhoea | 4 |

Bodyweight loss was calculated as the percent difference between the original bodyweight (Day 1) and the actual bodyweight on each experimental day (2-10).

The appearance of diarrhoea is defined as mucus/faecal material adherent to anal fur. Rectal bleeding is defined as diarrhoea containing visible blood/mucus or gross rectal bleeding. The maximum score of the DAI each day is 12.

Blood Sampling

Two blood samples were obtained from each animal on two separate occasions during the course of the study: on Day 1 and on Day 5. Blood samples were obtained on each occasion into Microvette CB-300 microtubes by puncture of the saphenous vein 2 hours after administration of the test item. This blood extraction method does not require anaesthetic or analgesics and produces a minimum stress in the animals (Hem et al., 1998). Additionally a terminal blood sample was obtained from all animals on the last day of the study from the abdominal vena cava also two hours after test item administration.

Blood samples were allowed to clot and then centrifuged at 3000 rpm for 10 min and the resulting serum frozen at -80°C for storage.

Euthanasia and Collection of Colon Samples

On day 10, two hours after the last administration of control vehicle, hBD2 or prednisolone, the animals were killed by an overdose of anaesthetic. Their colons were removed and their length and weight measured after exclusion of the caecum.

Two sections (proximal and distal) of colon were taken from each animal and preserved in neutral buffered formalin for subsequent histological analysis (haematoxylin and eosin staining) according to the following scoring system:

| Description | Score |
|---|---|
| No changes observed | 0 |
| Minimal scattered mucosal inflammatory cell infiltrates, with or without minimal epithelial hyperplasia. | 1 |
| Mild scattered to diffuse inflammatory cell infiltrates, sometimes extending into the submucosa and associated with erosions, with minimal to mild epithelial hyperplasia and minimal to mild mucin depletion from goblet cells. | 2 |
| Mild to moderate inflammatory cell infiltrates that were sometimes transmural, often associated with ulceration, with moderate epithelial hyperplasia and mucin depletion. | 3 |
| Marked inflammatory cell infiltrates that were often transmural and associated with ulceration, with marked epithelial hyperplasia and mucin depletion. | 4 |
| Marked transmural inflammation with severe ulceration and loss of intestinal glands. | 5 |

Determination of TNF-alpha Concentration in Colonic Tissue Samples

An additional sample of colon was obtained from each animal and homogenised in PBS (100 mg tissue/ml PBS) containing 1% bovine serum albumin (BSA) and a protease inhibitor cocktail (1ml/20g tissue). The homogenate was then be centrifuged at 1400 rpm for 10 min and the supernatant stored at -20°C for subsequent determination of TNF-$\alpha$ concentration by specific enzyme immunoassay (ELISA).

RESULTS

Disease Activity Index Score

Table 23. Disease Activity Index (DAI) score progression during Day 1 to Day 10. Significant differences from control (vehicle) group values at a given date are shown as *p<0.05; **p<0.01 (Kruskal-Wallis Test for non-parametric data).

| Test item | Data | DAI score | | | | |
|---|---|---|---|---|---|---|
| | | Day 1 | Day 2 | Day 3 | Day 4 | Day 5 |
| Group A Control vehicle i.v. | Mean | 0.00 | 1.10 | 1.30 | 3.20 | 2.90 |
| | S.E.M. | 0.00 | 0.31 | 0.37 | 0.36 | 0.31 |
| Group B hBD2 0.1 mg/kg i.v. | Mean | 0.00 | 0.20 | 0.80 | 2.90 | 2.80 |
| | S.E.M. | 0.00 | 0.13 | 0.20 | 0.10 | 0.13 |
| Group C hBD2 1 mg/kg i.v. | Mean | 0.00 | 0.00 | 0.22 | 2.22 | 2.44 |
| | S.E.M. | 0.00 | 0.00 | 0.22 | 0.15 | 0.18 |
| Group D hBD2 10 mg/kg i.v. | Mean | 0.00 | 0.60 | 1.00 | 3.67 | 3.11 |
| | S.E.M. | 0.00 | 0.22 | 0.44 | 0.24 | 0.26 |
| Group E Prednisolone 1 mg/kg p.o. | Mean | 0.00 | 0.10 | 0.00 | 2.60 | 2.50 |
| | S.E.M. | 0.00 | 0.10 | 0.00 | 0.22 | 0.22 |

Table 23 (continued).

| Test item | Data | DAI score | | | | |
|---|---|---|---|---|---|---|
| | | Day 6 | Day 7 | Day 8 | Day 9 | Day 10 |
| Group A Control vehicle i.v. | Mean | 3.10 | 4.10 | 5.90 | 8.90 | 10.90 |
| | S.E.M. | 0.31 | 0.69 | 1.26 | 1.02 | 0.62 |
| Group B hBD2 0.1 mg/kg i.v. | Mean | 3.20 | 1.44** | 2.11* | 3.89** | 6.44* |
| | S.E.M. | 0.20 | 0.38 | 0.20 | 0.35 | 0.85 |
| Group C hBD2 1 mg/kg i.v. | Mean | 2.89 | 2.22 | 3.67 | 5.22 | 6.44* |
| | S.E.M. | 0.20 | 0.43 | 0.80 | 0.83 | 1.08 |
| Group D hBD2 10 mg/kg i.v. | Mean | 3.22 | 2.11 | 4.11 | 6.78 | 7.33 |
| | S.E.M. | 0.28 | 0.31 | 0.93 | 1.20 | 1.33 |
| Group E Prednisolone 1 mg/kg p.o. | Mean | 2.60 | 3.10 | 2.50* | 3.80* | 4.90** |
| | S.E.M. | 0.27 | 0.96 | 0.43 | 0.98 | 0.91 |

Histological evaluation

Two sections (proximal and distal) of colon were taken from each animal, processed for histological analysis (haematoxylin and eosin staining) and scored by a blind observer according to the histological scoring system described above.

Determination of TNF-α concentration in colonic tissue samples

An additional sample of colon was obtained from each animal and homogenised in PBS (100 mg tissue/ml PBS) containing 1% bovine serum albumin (BSA) and a protease inhibitor cocktail (1ml/20g tissue). The homogenate was then be centrifuged at 14000 rpm for 10min and the supernatant stored at -20°C for subsequent determination of TNF-α concentration by specific enzyme immunoassay (ELISA).

Table 24. Histological scores, colon weight and length, and colon TNF-α concentration. Differences in histological scores from control (vehicle) group values are shown as *$p<0.05$; **$p<0.01$ (Kruskal-Wallis Test for non-parametric data).

| Test item | Data | Histology Score Proximal Colon | Histology Score Distal Colon | Colon TNF-α concentration (pg/g tissue) |
|---|---|---|---|---|
| Group A Control vehicle i.v. | Mean | **4.20** | **4.50** | **1664** |
| | S.E.M. | 0.25 | 0.22 | 227 |
| Group B hBD2 0.1 mg/kg i.v. | Mean | **2.22**** | **3.67** | **1185** |
| | S.E.M. | 0.43 | 0.47 | 205 |
| Group C hBD2 1 mg/kg i.v. | Mean | **2.89*** | **4.13** | **1457** |
| | S.E.M. | 0.35 | 0.35 | 211 |
| Group D hBD2 10 mg/kg i.v. | Mean | **2.89*** | **4.78** | **1212** |
| | S.E.M. | 0.39 | 0.15 | 211 |
| Group E Prednisolone 1 mg/kg p.o. | Mean | **2.80*** | **3.70** | **1833** |
| | S.E.M. | 0.51 | 0.42 | 414 |

STATISTICAL ANALYSIS

The statistical significance of the results was evaluated using the statistics program Graphpad Instat 3. The difference between groups for disease activity index and histological score was evaluated by Kruskal-Wallis test for unpaired data plus post-test Dunn to allow for multiple comparisons. A value of $p<0.05$ was taken as significant.

CONCLUSIONS

The results demonstrate that hBD2 at the lowest dose tested (0.1 mg/kg i.v.) significantly reduces the increase in Disease Activity Index induced by DSS administration at day 7 (1.44±0.38 test item vs. 4.1±0.69 vehicle; $p<0.01$), day 8 (2.11±0.2 test item vs. 5.9±1.26 vehicle; $p<0.05$), day 9 (3.89±0.35 test item vs. 8.9±1.02 vehicle; $p<0.01$) and day 10 (6.44±0.85 test item vs. 10.9±0.62 vehicle; $p<0.05$).
Treatment with the intermediate dose of hBD2 (1 mg/kg i.v.) for 10 consecutive days resulted in an apparent reduction of the disease activity index score but this was only significant on day 10 (6.44±1.08 test item vs. 10.9±0.62 vehicle; $p<0.05$).
Similarly to the results obtained with the Disease Activity Index on day 10, histological analysis of the proximal colons of each animal revealed a very significant reduction of histological damage score by treatment with the low dose of hBD2 (2.22±0.43 test item vs. 4.2±0.25 vehicle; $p<0.01$). Moreover, a significant reduction of histological injury was also observed with the intermediate and high doses of hBD2, as well as with prednisolone (2.89±0.35; 2.89±0.39 and 2.8±0.5 respectively; $p<0.05$). In contrast, in the distal colon - although an apparent reduction in histological injury could be observed in the animals treated with the low and intermediate dose of hBD2, as well as with prednisolone - this was not statistically significant. No reduction could be observed in the animals that were treated with the high dose of hBD2. Similarly, treatment with the low and intermediate doses of hBD2 resulted in an apparent reduction in colonic TNF-alpha levels, but this apparent reduction was not statistically significant.
The results obtained in the present study demonstrate an anti-inflammatory activity of hBD2 in the model of DSS colitis induced in the mouse after a 10-day treatment period. However, this anti-inflammatory activity appears to be more pronounced at the lower dose of hBD2 used (0.1 mg/kg/day i.v.) and is gradually lost with increasing doses up to the highest dose used in the study (10 mg/kg/day i.v.). Moreover, the anti-inflammatory effect of the lowest dose of hBD2 is comparable or even greater (e.g. histological score) than that of prednisolone at a dose of 1 mg/kg/day p.o.

**EXAMPLE 6**

**10-Day Dextran Sodium Sulphate (DSS)-induced colitis model in the mouse**

Example 6 was carried out essentially as described in Example 5. The differences are indicated below.

Weight

The average body weight of the animals on the day of start of the study was 19.74 $\pm$ 0.09 g (mean $\pm$ SEM).

Study design

Animals were divided into 9 experimental groups. Each group consisted of 10 males:

Group A: Treated with control vehicle (PBS) i.v.
Group B: Treated with hBD2 (1 mg/kg i.v.) - once daily
Group C: Treated with hBD2 (0.1 mg/kg i.v.) - once daily
Group D: Treated with hBD2 (0.01 mg/kg i.v.) - once daily
Group E: Treated with hBD2 (0.001 mg/kg i.v.) - once daily
Group F: Treated with hBD2 (0.1 mg/kg i.v. + s.c.) - twice daily
Group G: Treated with hBD2 (0.1 mg/kg i.v.) - every second day
Group H: Treated with methylprednisolone (1 mg/kg p.o.)
Group J: Treated with methylprednisolone (10 mg/kg p.o.)

Animal allocation to all experimental groups was done in a randomized manner. A maximum of 5 mice were housed in each cage (as per Directive 86/609/EEC). All animals were weighed on their arrival at the laboratory and prior to the administration of the test and reference compounds.

Administration of the test items

The control vehicle and hBD2 were administered intravenously via the tail vein with the use of a sterile needle (25G) in a dosing volume of 5 ml/kg body weight as a slow bolus (over a period of 15 seconds).
The animals in groups A to E received one dose daily (every 24 hours) of the corresponding test item (hBD2, prednisolone or control vehicle) for 10 consecutive days.
The animals in group F received one dose i.v. and another dose s.c. (12 hours after the i.v. dose) of the corresponding test item for 10 consecutive days.
The animals in group G received one dose every two days of the corresponding test item for 10 consecutive days.
Methylprednisolone was given orally at a dose of 1 mg/kg (group H) and 10 mg/kg (group J) in a dosing volume of 5 ml/kg body weight, once daily for 10 consecutive days.

Blood Sampling

A terminal blood sample was obtained from all animals on the last day of the study from the abdominal vena cava 2 hours after test item administration.
Blood samples were allowed to clot and then centrifuged at 3000 rpm for 10 min, and the resulting serum was frozen at -80°C for subsequent analysis.

RESULTS

Disease Activity Index Score

Table 25. Disease Activity Index (DAI) score progression during Day 1 to Day 10. Significant differences from control (vehicle) group values at a given date are shown as *p<0.05; **p<0.01 (Kruskal-Wallis Test for non-parametric data). Day 6 to Day 10 is shown on the next page.

| Test item | Data | DAI score | | | | |
|---|---|---|---|---|---|---|
| | | Day 1 | Day 2 | Day 3 | Day 4 | Day 5 |
| Group A Control vehicle i.v. | Mean | 0.00 | 0.00 | 0.10 | 0.10 | 0.20 |
| | S.E.M. | 0.00 | 0.00 | 0.10 | 0.10 | 0.13 |
| Group B hBD2 1 mg/kg i.v. | Mean | 0.00 | 0.10 | 0.20 | 0.40 | 0.30 |
| | S.E.M. | 0.00 | 0.10 | 0.13 | 0.16 | 0.21 |
| Group C hBD2 0.1 mg/kg i.v. | Mean | 0.00 | 0.44 | 0.89 | 0.56 | 0.78 |
| | S.E.M. | 0.00 | 0.18 | 0.42 | 0.29 | 0.28 |
| Group D hBD2 0.01 mg/kg i.v. | Mean | 0.00 | 0.00 | 0.30 | 0.40 | 1.60 |
| | S.E.M. | 0.00 | 0.00 | 0.15 | 0.16 | 0.43 |
| Group E hBD2 0.001 mg/kg i.v. | Mean | 0.00 | 0.00 | 0.10 | 0.20 | 0.40 |
| | S.E.M. | 0.00 | 0.00 | 0.10 | 0.13 | 0.16 |
| Group F hBD2 0.1 mg/kg twice daily i.v.+s.c. | Mean | 0.00 | 0.30 | 0.70 | 0.70 | 0.60 |
| | S.E.M. | 0.00 | 0.21 | 0.30 | 0.34 | 0.16 |
| Group G hBD2 0.1 mg/kg i.v. every 2. day | Mean | 0.00 | 0.20 | 0.40 | 0.50 | 0.50 |
| | S.E.M. | 0.00 | 0.13 | 0.22 | 0.17 | 0.17 |
| Group H Prednisolone 1 mg/kg p.o. | Mean | 0.00 | 0.50 | 0.50 | 0.40 | 1.10 |
| | S.E.M. | 0.00 | 0.17 | 0.17 | 0.16 | 0.18 |
| Group J Prednisolone 10 mg/kg p.o. | Mean | 0.00 | 0.30 | 0.70 | 0.80 | 1.30 |
| | S.E.M. | 0.00 | 0.15 | 0.21 | 0.20 | 0.21 |

Table 25 (continued).

| Test item | Data | DAI score | | | | |
|---|---|---|---|---|---|---|
| | | Day 6 | Day 7 | Day 8 | Day 9 | Day 10 |
| Group A Control vehicle i.v. | Mean | **6.90** | **9.67** | **11.11** | **11.67** | **11.00** |
| | S.E.M. | 1.02 | 0.33 | 0.31 | 0.17 | 0.65 |
| Group B hBD2 1 mg/kg i.v. | Mean | **2.30*** | **4.40*** | **6.89** | **5.00*** | **5.78*** |
| | S.E.M. | 1.00 | 1.03 | 1.41 | 0.60 | 0.70 |
| Group C hBD2 0.1 mg/kg i.v. | Mean | **1.56**** | **4.13*** | **5.43*** | **6.29*** | **6.86** |
| | S.E.M. | 0.73 | 0.83 | 1.13 | 1.64 | 1.14 |
| Group D hBD2 0.01 mg/kg i.v. | Mean | **2.70** | **6.50** | **6.20*** | **4.60**** | **5.20**** |
| | S.E.M. | 1.08 | 1.28 | 1.06 | 0.98 | 0.87 |
| Group E hBD2 0.001 mg/kg i.v. | Mean | **3.40** | **7.11** | **8.56** | **5.89**** | **6.67** |
| | S.E.M. | 1.32 | 1.38 | 1.06 | 1.63 | 1.30 |
| Group F hBD2 0.1 mg/kg twice daily i.v.+s.c. | Mean | **0.70**** | **3.50**** | **4.00**** | **2.90**** | **4.50**** |
| | S.E.M. | 0.30 | 0.89 | 1.17 | 0.55 | 0.62 |
| Group G hBD2 0.1 mg/kg i.v. every 2. day | Mean | **2.90** | **6.50** | **8.70** | **7.50** | **6.56** |
| | S.E.M. | 1.12 | 1.11 | 1.25 | 0.93 | 0.99 |
| Group H Prednisolone 1 mg/kg p.o. | Mean | **3.80** | **5.90** | **6.40** | **5.60*** | **5.60*** |
| | S.E.M. | 0.98 | 1.16 | 0.88 | 0.88 | 0.65 |
| Group J Prednisolone 10 mg/kg p.o. | Mean | **2.00** | **3.20**** | **4.80**** | **5.20*** | **4.00**** |
| | S.E.M. | 0.30 | 0.73 | 0.53 | 0.61 | 0.00 |

Histological evaluation

Two sections (proximal and distal) of colon were taken from each animal, processed for histological analysis (haematoxylin and eosin staining), and scored by a blind observer according to the scoring system described above.

Table 26. Histological scores, colon weight and length, and colon TNF-$\alpha$ concentration. Differences in histological scores from control (vehicle) group values are shown as *p<0.05; **p<0.01 (Kruskal-Wallis Test for non-parametric data).

| Test item | Data | Histology Score Proximal Colon | Histology Score Distal Colon |
|---|---|---|---|
| Group A Control vehicle i.v. | Mean | **2.44** | **4.67** |
| | S.E.M. | 0.34 | 0.17 |
| Group B hBD2 1 mg/kg i.v. | Mean | **1.78** | **3.56** |
| | S.E.M. | 0.36 | 0.38 |
| Group C | Mean | **1.71** | **3.14*** |

(continued)

| Test item | Data | Histology Score Proximal Colon | Histology Score Distal Colon |
|---|---|---|---|
| hBD2 0.1 mg/kg i.v. | S.E.M. | 0.18 | 0.40 |
| Group D hBD2 0.01 mg/kg i.v. | Mean | **1.70** | **3.10\*\*** |
| | S.E.M. | 0.26 | 0.23 |
| Group E hBD2 0.001 mg/kg i.v. | Mean | **1.44** | **3.56** |
| | S.E.M. | 0.24 | 0.18 |
| Group F hBD2 0.1 mg/kg twice daily i.v.+s.c. | Mean | **1.30\*** | **2.90\*\*\*** |
| | S.E.M. | 0.21 | 0.23 |
| Group G hBD2 0.1 mg/kg i.v. every 2. day | Mean | **1.56** | **3.56** |
| | S.E.M. | 0.24 | 0.29 |
| Group H Prednisolone 1 mg/kg p.o. | Mean | **1.40** | **3.00\*\*\*** |
| | S.E.M. | 0.22 | 0.00 |
| Group J Prednisolone 10 mg/kg p.o. | Mean | **1.40** | **2.70\*\*\*** |
| | S.E.M. | 0.16 | 0.21 |

STATISTICAL ANALYSIS

The statistical significance of the results was evaluated using the statistics program Graphpad Instat 3. The difference between groups for disease activity index and histological score was evaluated by Kruskal-Wallis test for unpaired data + post-test of Dunn for multiple comparisons. A value of $p < 0.05$ was taken as significant. In the tables above, significant differences versus the corresponding control (vehicle) group are denoted as: *$p < 0.05$, **$p < 0.01$, ***$p < 0.001$.

CONCLUSIONS

The aim of the present study was to determine the anti-inflammatory activity of hBD2 in an acute (10-days) model of inflammatory bowel disease (colitis) induced by oral dextran sodium sulphate (DSS, 2%) administration in the mouse.

The results obtained in the present study further demonstrate an anti-inflammatory activity of hBD2 in the model of DSS colitis induced in the mouse after a 10-day treatment period.

This anti-inflammatory activity appears to be more pronounced after administration of hBD2 twice per day (every 12 hours), both intravenously and subcutaneously, at a dose of 0.1 mg/kg. Moreover, the anti-inflammatory effect observed with this dose of hBD2 is comparable, or even greater (both on Disease Activity Index and histological score), than that of prednisolone at a dose of 1 mg/kg or 10 mg/kg given orally.

**EXAMPLE 7**

**Evaluation of Human Beta Defensin 2 in a Collagen-Induced Rheumatoid Arthritis Model**

The aim of the following study was to determine the anti-inflammatory activity of human beta defensin 2 in a collagen-induced rheumatoid arthritis model in the mouse.

TEST SYSTEM

Species/Strain: Mouse / DBA/1
Source: Harlan, UK
Gender: Male
No. of Animals: *n = 50*

(continued)

| | |
|---|---|
| Age: | Young adults, 6-8 weeks of age at study initiation. |
| Body Weight: | Weight variation of study animals at the time of collagen induction did not exceed ± 20% of the mean weight. |
| Animals Health: | The health status of the animals used in this study was examined on arrival. Only animals in good health were acclimatized to laboratory conditions and were used in the study. |
| Acclimatization: | At least 7 days. |
| Housing: | During acclimatization and following dosing, animals were housed within a limited access rodent facility and kept in groups of maximum 10 mice, in polypropylene cages (45 cm x 25 cm x 13 cm), fitted with solid bottoms and filled with wood shavings as bedding material. Cages were changed once weekly. |
| Food and Water: | Animals were provided *ad libitum* a commercial rodent diet and free access to drinking water, supplied to each cage via polyethylene bottles with stainless steel sipper tubes. Water bottles were changed at least every 3 weeks. Water was changed 3 times per week. |
| Environment: | Automatically controlled environmental conditions were set to maintain temperature at 20-24°C with a relative humidity (RH) of 30-70%, a 12/12 hour light/dark cycle and 10-30 air changes/hr in the study room. Temperature and RH was monitored daily by both manual measurements and the control computer. The light cycle was monitored by the control computer. |
| Identification: | Animals were given a unique animal identification ear number. This number also appeared on a cage card, visible on the front of each cage. The cage card also contained the study number. |
| Randomization: | Animals were randomly assigned to experimental groups. |
| Termination: | At the end of the study surviving animals were euthanized by $O_2/CO_2$ inhalation, followed by exsanguination. |
| Justification: | The mouse was selected since it represented the species of choice for this experimental animal model. The DBA/1 strain of mouse is highly susceptible to collagen-induced arthritis (CIA). |

MATERIALS

Human Beta Defensin 2 (hBD2); see Example 1
Dexamethasone (Sigma, cat. no. D1756)
Bovine Type II Collagen (MD Biosciences, cat. no. 804001314)
Complete Freund's Adjuvant (CFA) (MD Biosciences, cat. no. 501009703)
PBS (PAA, cat no. H15-002)

CONSTITUTION OF TEST GROUPS

Table 27. Test groups and treatments.

| Group size | Group no. | Test compound | Route | Dose | Volume | Regime |
|---|---|---|---|---|---|---|
| n=10 | A | Vehicle control | IV | 0 mg/kg | 5 mL/kg | once daily |
| n=10 | B | Dexamethasone | IP | 1 mg/kg | 5 mL/kg | once daily |
| n=10 | C | hBD2 | IV | 10 mg/kg | 5 mL/kg | once daily |
| n=10 | D | hBD2 | IV | 1 mg/kg | 5 mL/kg | once daily |
| n=10 | E | hBD2 | IV | 0.1 mg/kg | 5 mL/kg | once daily |
| IV: intravenous IP: intraperitoneal | | | | | | |

TEST PROCEDURES

Arthritis Induction

All animals were subjected on Day 0 of the study (study commencement) to an intradermal injection into the tail of 0.1 ml Type II Collagen/CFA emulsion (200 μg collagen per mouse) under light Isoflurane anesthesia, using a plastic syringe. The location of injection was at an approximate caudal distance of about 1 cm from the base of the tail. A collagen challenge (200 μg/mouse) was presented to the animals by IP injection of collagen and PBS on Day 21.

Treatment

Treatments were commenced on day 14 of the study and continued once daily throughout. All surviving mice were terminated on study day 42.

Route of Administration:

| (i) | hBD2: | Intravenous |
|-----|-------|-------------|
| (ii) | Dexamethasone: | Intraperitoneal |
| (iii) | Vehicle Control: | Intravenous |

Dose and Volume Dosage (see also Table 27):

| (i) | hBD2: | 10, 1 or 0.1 mg/kg at 5 mL/kg |
|-----|-------|-------------------------------|
| (ii) | Dexamethasone: | 1 mg/kg at 5 mL/kg |
| (iii) | Vehicle Control: | 0 mg/kg at 5 mL/kg |

Analgesia: No analgesic was used during the study.

OBSERVATIONS AND EXAMINATIONS

Arthritis Reactions

Mice were examined for signs of arthritogenic responses in peripheral joints on study day 0, 14, 21 and thereafter five times weekly until termination of the study. Arthritis reactions were reported for each paw according to a 0-4 scale in ascending order of severity as shown below:

| Arthritis Score | Grade |
|-----------------|-------|
| No reaction, normal: | 0 |
| Mild, but definite redness and swelling of the ankle/wrist or apparent redness and swelling limited to individual digits, regardless of the number of affected digits: | 1 |
| Moderate to severe redness and swelling of the ankle/wrist: | 2 |
| Redness and swelling of the entire paw including digits: | 3 |
| Maximally inflamed limb with involvement of multiple joints: | 4 |

Clinical Signs

On Day 0, 14, 21 and thereafter five times weekly, careful clinical examinations were carried out and recorded. Observations included changes in skin, fur, eyes, mucous membranes, occurrence of secretions and excretions (e.g. diarrhea) and autonomic activity (e.g. lacrimation, salivation, piloerection, pupil size, unusual respiratory pattern). Changes in gait, posture and response to handling, as well as the presence of bizarre behavior, tremors, convulsions, sleep and coma were also noted.

Prior to day 14 mice were monitored daily for any unusual behaviour.

Body Weights

Determination of individual body weights of animals were made shortly before Arthritis induction on Day 0, 14, 21 and thereafter five times weekly until the termination of the study.

Measurement of Experimental Arthritis

The relative change in both hind paw thickness (left and right, just below the foot pad and above the calcaneum) of each animal was measured in mm on study days 0, 14, 21 and thereafter five times per week using a dial caliper (Kroeplin, Munich, Germany).

Study Termination

All mice were terminated on study day 42.

Sample Collection

At study termination, following $O_2/CO_2$ inhalation, terminal blood samples were obtained from all remaining study animals. Serum was prepared from each sample and stored at -20°C. In addition, the left front and rear paws were collected and stored in formalin, and the right front and rear paws were collected and snap frozen for possible joint RNA analysis.

Humane Endpoints

Animals found in a moribund condition and animals showing severe pain and enduring signs of severe distress were humanely euthanized. In addition, animals showing a decrease of body weight larger than 20% from initial body weight determination were humanely euthanized. Mice with a total arthritic score of 12 or higher were also culled for humane reasons. All animals were euthanized by $O_2/CO_2$ inhalation, followed by exsanguination. Paw samples and terminal blood samples were obtained from all study animals.

STATISTICAL ANALYSIS

Evaluation was primarily based on the mean values for arthritis scores and paw thickness measurements. Where appropriate, analysis of the data by appropriate statistical methods was applied to determine significance of treatment effects. ANOVA followed by Tukey post-hoc analysis (Winstat 2005.1 for Excel) was used to assess statistical differences between treatment groups.

In accordance with Home Office regulations mice with a total clinical score of equal to or greater than 12 were culled due to arthritis severity. The clinical score of these mice at termination was carried forward in the analysis for the remainder of the study in order that the data was not artificially skewed by the removal of high scoring mice.

ANIMAL CARE AND USE STATEMENT

This study was performed according to the UK Home Office regulations for use of animals in scientific procedures.

RESULTS

Table 28. Mean clinical arthritis scores determined during the 42 day observation period in the collagen induced male DBA/1 arthritic mice. * p<0.05 significantly different from Vehicle group

| Study Day | Data | Group A Vehicle | Group B Dexamethasone 1 mg/kg | Group C hBD2 10 mg/kg | Group D hBD2 1 mg/kg | Group E hBD2 0.1 mg/kg |
|---|---|---|---|---|---|---|
| 0 | Mean Arthritic Score | 0.0 | 0.0 | 0.0 | 0.0 | 0.0 |
| | SEM | 0.0 | 0.0 | 0.0 | 0.0 | 0.0 |
| 14 | Mean Arthritic Score | 0.1 | 0.4 | 1.7 | 0.6 | 1.2 |
| | SEM | 0.1 | 0.2 | 0.6 | 0.3 | 0.5 |
| 21 | Mean Arthritic Score | 3.2 | 0.0 | 3.1 | 2.1 | 3.2 |
| | SEM | 1.2 | 0.0 | 1.1 | 0.8 | 1.3 |
| 22 | Mean Arthritic Score | 3.4 | 0.0 | 4.3 | 2.3 | 3.6 |
| | SEM | 1.1 | 0.0 | 1.1 | 0.8 | 1.4 |
| 23 | Mean Arthritic Score | 3.4 | 0.0 | 3.3 | 2.0 | 3.2 |
| | SEM | 1.1 | 0.0 | 1.2 | 0.7 | 1.3 |
| 26 | Mean Arthritic Score | 4.4 | 0.0* | 4.1 | 2.0 | 3.7 |
| | SEM | 1.1 | 0.0 | 1.2 | 0.7 | 1.3 |
| 27 | Mean Arthritic Score | 4.8 | 0.0* | 4.1 | 2.0 | 4.1 |
| | SEM | 1.2 | 0.0 | 1.3 | 0.7 | 1.3 |
| 28 | Mean Arthritic Score | 5.3 | 0.0* | 4.1 | 2.3 | 4.4 |
| | SEM | 1.1 | 0.0 | 1.2 | 0.8 | 1.3 |
| 29 | Mean Arthritic Score | 6.3 | 0.0* | 4.4 | 2.4 | 5.0 |
| | SEM | 1.1 | 0.0 | 1.3 | 0.8 | 1.3 |
| 30 | Mean Arthritic Score | 6.8 | 0.0* | 4.9 | 2.7 | 5.9 |
| | SEM | 1.1 | 0.0 | 1.3 | 0.9 | 1.5 |
| 33 | Mean Arthritic Score | 7.4 | 0.0* | 4.8 | 4.0 | 7.3 |
| | SEM | 1.1 | 0.0 | 1.2 | 0.9 | 1.4 |

(continued)

| Study Day | Data | Group A Vehicle | Group B Dexamethasone 1 mg/kg | Group C hBD2 10 mg/kg | Group D hBD2 1 mg/kg | Group E hBD2 0.1 mg/kg |
|---|---|---|---|---|---|---|
| 34 | Mean Arthritic Score | **7.3** | **0.0\*** | **4.9** | **4.1** | **7.8** |
| | SEM | 1.1 | 0.0 | 1.2 | 1.1 | 1.3 |
| 35 | Mean Arthritic Score | **7.5** | **0.0\*** | **4.9** | **4.0** | **8.0** |
| | SEM | 1.0 | 0.0 | 1.2 | 1.1 | 1.3 |
| 36 | Mean Arthritic Score | **6.8** | **0.0\*** | **4.8** | **4.1** | **8.2** |
| | SEM | 0.9 | 0.0 | 1.1 | 1.1 | 1.1 |
| 37 | Mean Arthritic Score | **7.4** | **0.0\*** | **4.7** | **4.2** | **8.7** |
| | SEM | 0.9 | 0.0 | 1.1 | 1.1 | 1.1 |
| 40 | Mean Arthritic Score | **8.2** | **0.0\*** | **5.0** | **4.7\*** | **9.0** |
| | SEM | 0.8 | 0.0 | 1.0 | 1.2 | 0.8 |
| 41 | Mean Arthritic Score | **8.5** | **0.0\*** | **4.8\*** | **5.2** | **8.8** |
| | SEM | 0.7 | 0.0 | 1.0 | 1.1 | 0.8 |

CONCLUSION

Arthritic reactions were noted in all groups from study day 14. Mean total arthritis scores (Table 28) for vehicle treated mice peaked at 8.5 $\pm$ 0.72 on study day 41. Mean total arthritis scores in mice treated with hBD2 at 10 mg/kg (Group C) peaked at 5.0 $\pm$ 1.04 on study day 40. Mean arthritis scores in this group were lower compared to vehicle treated mice from day 23 until the end of study, however only significantly on study day 41.

Mean total arthritis scores in mice treated with hBD2 at 1 mg/kg (Group D) peaked at 5.2 $\pm$ 1.11 on study day 41 and were consistently lower compared to the vehicle treated group from day 21 until the end of study, however only significantly on day 40. Treatment of mice with hBD2 at 0.1 mg/kg (Group E) did not significantly lower mean total arthritis scores compared to the vehicle treated group. The mean score in this group peaked at 9.0 $\pm$ 0.77 on study day 40. Mice in the dexamethasone treated group (Group B) displayed a significantly lower arthritic score compared to the vehicle treated group from study day 26 until the end of the study.

To ensure that the removal of mice culled early in the study due to arthritis severity did not artificially skew the data, arthritis scores from such mice were carried over in the analysis until study termination.

REFERENCES

Bonoiotto M., WJ Jordan, J. Eskdale, A. Tossi, N. Antcheva, S. Crovella, ND Connell and G Gallagher. Human β-Defensin 2 Induces a Vigorous Cytokine Response in Peripheral Blood Mononuclear Cells. Antimicrobial Agents and Chemotherapy (2006), 50, 1433-1441.

Bowdish et al., Immunomodulatory properties of defensins and cathelicidins. Curr. Top. Microbiol. Immunol. (2006) 306, 27-66.

Gersemann et al., Crohn's disease--defect in innate defence. World J. Gastroenterol. (2008) 14, 5499-5503.

Lehrer R.I., Primate defensins. Nat. Rev. Microbiol. (2004) 2, 727-738.

Swidsinski et al., Mucosal flora in inflammatory bowel disease. Gastroenterology (2002) 122, 44-54.

Niyonsaba F., H. Ushio, N. Nakano, W. Ng, K. Sayama, K. Hashimoto, I. Nagaoka, K. Okumura and H. Ogawa. Antimicrobial peptides human β-defensins stimulate epidermal keratinocyte migration, proliferation and production of proinflammatory cytokines and chemokines. Journal of Investigative Dermatology (2007), 127, 594-604.

Rowland TL, SM McHugh, J Deighton, RJ Dearman, PW Ewan and I Kimber. Differential regulation by thalidomide and dexamethasone of cytokine expression in human peripheral blood mononuclear cells. Immunopharmacology (1998), 40, 11-20.

Wang et al., Host-microbe interaction: mechanisms of defensin deficiency in Crohn's disease. Expert. Rev. Anti. Infect. Ther. (2007) 5, 1049-1057.

Wehkamp et al., Reduced Paneth cell alpha-defensins in ileal Crohn's disease. Proc. Natl. Acad. Sci. U. S. A (2005) 102, 18129-18134.

**Claims**

1. Use of a human beta defensin having at least 80% identity to the amino acid sequences of SEQ ID NO:1, SEQ ID NO:2, SEQ ID NO:3 or SEQ ID NO:4 in the manufacture of a medicament for the treatment of an inflammatory disease or disorder selected from the group consisting of arthritis, multiple sclerosis, atherosclerosis, scleroderma (systemic sclerosis), lupus, systemic lupus erythematosus (SLE), (acute) glomerulonephritis, asthma, inflammatory bowel disease, ulcerative colitis, vasculitis, uveitis, dermatitis, atopic dermatitis, alopecia, rhinitis (allergica), myasthenia gravis, sclerodermitis, sarcoidosis, psoriatic arthritis, psoriasis, ankylosing spondylitis, Graves disease, Sjogren's syndrome, and Behçet disease.

2. A human beta defensin having at least 80% identity to the amino acid sequences of SEQ ID NO:1, SEQ ID NO:2, SEQ ID NO:3 or SEQ ID NO:4 for use in the treatment of an inflammatory disease or disorder selected from the group consisting of arthritis, artherosclerosis, scleroderma (systemic sclerosis), lupus, systemic lupus erythematosus (SLE), (acute) glomerulonephritis, asthma, inflammatory bowel disease, ulcerative colitis, vasculitis, uveitis, dermatitis, atopic dermatitis, alopecia, rhinitis (allergica), myasthenia gravis, sclerodermitis, sarcoidosis, psoriatic arthritis, psoriasis, ankylosing spondylitis, Graves disease, Sjogren's syndrome, and Behçet disease.

3. The use or the human defensin for the use according to any one of claims 1 and 2, wherein the inflammatory bowel disease is Crohn's Disease.

4. The use or the human defensin for the use according to any one of claims 1 and 2, wherein the dermatitis is inflammatory dermatitis.

5. The use or the human defensin for the use according to any one of claims 1 and 2, wherein the arthritis is selected from rheumatoid arthritis, osteoarthritis, psoriatic arthritis, and juvenile idiopathic arthritis.

6. The use or the human beta defensin for the use according to any one of claims 1 to 5, wherein the medicament or the human beta defensin is administered enterally, parenterally, topically or as part of a sustained release implant.

7. The use or the human beta defensin for the use according to claim 6, wherein the parenteral administration is subcutaneous, intravenous, intraperitoneal, intracranial or intramuscular administration.

8. The use or the human beta defensin for the use according to claim 6, wherein the enteral administration is buccal, oral, nasal or rectal administration.

9. The use or the human beta defensin for the use according to claim 6, wherein the topical administration is epicutaneous, intranasal, or intratracheal.

10. The use or the human beta defensin for the use according to any one of claims 1-9, wherein the human beta defensin is administered at a daily dosage of from 0.001 mg/kg body weight to 10 mg/kg body weight, preferably from 0.01 mg/kg body weight to 10 mg/kg body weight.

11. The use or the human beta defensin for the use according to any one of claims 1-10, wherein the human beta defensin is human beta defensin 1, human beta defensin 2, human beta defensin 3, or human beta defensin 4.

12. The use or the human beta defensin for the use according to any of claims 1-11, wherein the human beta defensin has at least 80% identity to the amino acid sequence of SEQ ID NO:2.

13. The use or the human beta defensin for the use according to any one of claims 1-12, wherein the human beta defensin is human beta defensin 2.

14. The use or the human beta defensin for the use according to any one of claims 1-13, wherein TNF-alpha activity is reduced in the treated tissues.

**Patentansprüche**

1. Verwendung eines menschlichen beta-Defensins mit mindestens 80 % Identität zu den Aminosäuresequenzen der SEQ ID NO:1, SEQ ID NO:2, SEQ ID NO:3 oder SEQ ID NO:4 zur Herstellung eines Medikaments zur Behandlung einer entzündlichen Erkrankung oder Störung, die ausgewählt ist aus der Gruppe bestehend aus Arthritis, Multipler Sklerose, Atherosklerose, Sklerodermie (systemische Sklerose), Lupus, systemischem Lupus erythematodes (SLE), (akuter) Glomerulonephritis, Asthma, entzündlicher Darmerkrankung, ulzeröser Kolitis, Vaskulitis, Uveitis, Dermatitis, atopischer Dermatitis, Alopezie, Rhinitis (Allergien), Myasthenia gravis, Sklerodermitis, Sarkoidose, psoriatische Arthritis, Psoriasis, Ankylose-Spondylitis, Morbus Basedow, Sjögren-Syndrom und Morbus Behçet.

2. Menschliches beta-Defensin mit mindestens 80 % Identität zu den Aminosäuresequenzen der SEQ ID NO:1, SEQ ID NO:2, SEQ ID NO:3 oder SEQ ID NO:4 zur Verwendung bei der Behandlung einer entzündlichen Erkrankung oder Störung, die ausgewählt ist aus der Gruppe bestehend aus Arthritis, Arteriosklerose, Sklerodermie (systemische Sklerose), Lupus, systemischem Lupus erythematodes (SLE), (akuter) Glomerulonephritis, Asthma, entzündlicher Darmerkrankung, ulzeröser Kolitis, Vaskulitis, Uveitis, Dermatitis, atopischer Dermatitis, Alopezie, Rhinitis (Allergien), Myasthenia gravis, Sklerodermitis, Sarkoidose, psoriatische Arthritis, Psoriasis, Ankylose-Spondylitis, Morbus Basedow, Sjögren-Syndrom und Morbus Behçet.

3. Verwendung oder menschliches Defensin zur Verwendung nach einem der Ansprüche 1 und 2, wobei die entzündliche Darmerkrankung Morbus Crohn ist.

4. Verwendung oder menschliches Defensin zur Verwendung nach einem der Ansprüche 1 und 2, wobei die Dermatitis eine entzündliche Dermatitis ist.

5. Verwendung oder menschliches Defensin zur Verwendung nach einem der Ansprüche 1 und 2, wobei die Arthritis ausgewählt ist aus rheumatoider Arthritis, Osteoarthritis, psoriatischer Arthritis und juveniler idiopathischer Arthritis.

6. Verwendung oder menschliches beta-Defensin zur Verwendung nach einem der Ansprüche 1 bis 5, wobei das Medikament oder das menschliche beta-Defensin enteral, parenteral, topisch oder als Teil eines Implantats zur verzögerten Freisetzung verabreicht wird.

7. Verwendung oder menschliches beta-Defensin zur Verwendung nach Anspruch 6, wobei die parenterale Verabreichung eine subkutane, intravenöse, intraperitoneale, intrakranielle oder intramuskuläre Verabreichung ist.

8. Verwendung oder menschliches beta-Defensin zur Verwendung nach Anspruch 6, wobei die enterale Verabreichung eine bukkale, orale, nasale oder rektale Verabreichung ist.

9. Verwendung oder menschliches beta-Defensin zur Verwendung nach Anspruch 6, wobei die topische Verabreichung epikutan, intranasal oder intratracheal ist.

10. Verwendung oder menschliches beta-Defensin zur Verwendung nach einem der Ansprüche 1-9, wobei das menschliche beta-Defensin in einer Tagesdosis von 0,001 mg/kg Körpergewicht bis 10 mg/kg Körpergewicht, vorzugsweise von 0,01 mg/kg Körpergewicht bis 10 mg/kg Körpergewicht, verabreicht wird.

11. Verwendung oder menschliches beta-Defensin zur Verwendung nach einem der Ansprüche 1-10, wobei das menschliche beta-Defensin menschliches beta-Defensin 1, menschliches beta-Defensin 2, menschliches beta-Defensin 3 oder menschliches beta-Defensin 4 ist.

12. Verwendung oder menschliches beta-Defensin zur Verwendung nach einem der Ansprüche 1-11, wobei das menschliche beta-Defensin mindestens 80 % Identität mit der Aminosäuresequenz der SEQ ID N0:2 aufweist.

**13.** Verwendung oder menschliches beta-Defensin zur Verwendung nach einem der Ansprüche 1-12, wobei das menschliche beta-Defensin menschliches beta-Defensin 2 ist.

**14.** Verwendung oder menschliches beta-Defensin zur Verwendung nach einem der Ansprüche 1-13, wobei die TNF-alpha-Aktivität in den behandelten Geweben reduziert ist.

**Revendications**

**1.** Utilisation d'une bêta-défensine humaine ayant au moins 80 % d'identité avec les séquences d'acides aminés SEQ ID NO:1, SEQ ID NO:2, SEQ ID NO:3 ou SEQ ID NO:4 dans la fabrication d'un médicament pour le traitement d'une maladie ou d'un trouble inflammatoire, choisi(e) dans le groupe constitué de l'arthrite, de la sclérose en plaques, de l'athérosclérose, de la sclérodermie (sclérose systémique), du lupus, du lupus érythémateux systémique (SLE), de la glomérulonéphrite (aiguë), de l'asthme, de la maladie inflammatoire de l'intestin, de la colite ulcéreuse, de la vascularite, de l'uvéite, de la dermatite, de la dermatite atopique, de l'alopécie, de la rhinite (allergique), de la myasthénie grave, de la sclérodermite, de la sarcoïdose, de l'arthrite psoriasique, du psoriasis, de la spondylarthrite ankylosante, de la maladie de Graves, du syndrome de Sjogren et de la maladie de Behçet.

**2.** Bêta-défensine humaine ayant au moins 80 % d'identité avec les séquences d'acides aminés SEQ ID NO:1, SEQ ID NO:2, SEQ ID NO:3 ou SEQ ID NO:4 pour son utilisation dans le traitement d'une maladie ou d'un trouble inflammatoire, choisi(e) dans le groupe constitué de l'arthrite, de l'athérosclérose, de la sclérodermie (sclérose systémique), du lupus, du lupus érythémateux systémique (SLE), de la glomérulonéphrite (aiguë), de l'asthme, de la maladie inflammatoire de l'intestin, de la colite ulcéreuse, de la vascularite, de l'uvéite, de la dermatite, de la dermatite atopique, de l'alopécie, de la rhinite (allergique), de la myasthénie grave, de la sclérodermite, de la sarcoïdose, de l'arthrite psoriasique, du psoriasis, de la spondylarthrite ankylosante, de la maladie de Graves, du syndrome de Sjogren et de la maladie de Behçet.

**3.** Utilisation ou défensine humaine pour l'utilisation selon l'une quelconque des revendications 1 et 2, dans laquelle la maladie inflammatoire de l'intestin est la maladie de Crohn.

**4.** Utilisation ou défensine humaine pour l'utilisation selon l'une quelconque des revendications 1 et 2, dans laquelle la dermatite est la dermatite inflammatoire.

**5.** Utilisation ou défensine humaine pour l'utilisation selon l'une quelconque des revendications 1 et 2, dans laquelle l'arthrite est choisie parmi l'arthrite rhumatoïde, l'ostéoarthrite, l'arthrite psoriasique et l'arthrite juvénile idiopathique.

**6.** Utilisation ou bêta-défensine humaine pour l'utilisation selon l'une quelconque des revendications 1 à 5, dans laquelle le médicament ou la bêta-défensine humaine est administré(e) par voie entérale, parentérale, topique ou dans le cadre d'un implant à libération prolongée.

**7.** Utilisation ou bêta-défensine humaine pour l'utilisation selon la revendication 6, dans laquelle l'administration parentérale est une administration sous-cutanée, intraveineuse, intrapéritonéale, intracrânienne ou intramusculaire.

**8.** Utilisation ou bêta-défensine humaine pour l'utilisation selon la revendication 6, dans laquelle l'administration entérale est une administration buccale, orale, nasale ou rectale.

**9.** Utilisation ou bêta-défensine humaine pour l'utilisation selon la revendication 6, dans laquelle l'administration topique est épicutanée, intranasale ou intratrachéale.

**10.** Utilisation ou bêta-défensine humaine pour l'utilisation selon l'une quelconque des revendications 1 à 9, dans laquelle la bêta-défensine humaine est administrée à une dose journalière de 0,001 mg/kg de poids corporel à 10 mg/kg de poids corporel, de préférence de 0,01 mg/kg de poids corporel à 10 mg/kg de poids corporel.

**11.** Utilisation ou bêta-défensine humaine pour l'utilisation selon l'une quelconque des revendications 1 à 10, dans laquelle la bêta-défensine humaine est la bêta-défensine humaine 1, la bêta-défensine humaine 2, la bêta-défensine humaine 3 ou la bêta-défensine humaine 4.

**12.** Utilisation ou bêta-défensine humaine pour l'utilisation selon l'une quelconque des revendications 1 à 11, dans laquelle la bêta-défensine humaine a au moins 80 % d'identité avec la séquence d'acides aminés de SEQ ID NO:2.

**13.** Utilisation ou bêta-défensine humaine pour l'utilisation selon l'une quelconque des revendications 1 à 12, dans laquelle la bêta-défensine humaine est la bêta-défensine humaine 2.

**14.** Utilisation ou bêta-défensine humaine pour l'utilisation selon l'une quelconque des revendications 1 à 13, dans laquelle l'activité du TNF-alpha est réduite dans les tissus traités.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007081486 A
- WO 2007007116 A
- WO 9517413 A
- WO 9522625 A
- US 5223409 A
- WO 9206204 A
- WO 9802441 A
- WO 0114387 A
- WO 0364383 A
- AP 23573
- WO 0238561 A
- WO 0382859 A
- WO 2004052359 A
- WO 2005066156 A

**Non-patent literature cited in the description**

- **NEEDLEMAN ; WUNSCH.** *J. Mol. Biol.,* 1970, vol. 48, 443-453
- **RICE et al.** The European Molecular Biology Open Software Suite. *Trends in Genetics,* 2000, vol. 16, 276-277, http://emboss.org
- **RICE et al.** *EMBOSS: The European Molecular Biology Open Software Suite,* 2000, http://emboss.org
- **H. NEURATH ; R.L. HILL.** In, The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochem.,* 1991, vol. 30, 10832-10837
- **DERBYSHIRE et al.** *Gene,* 1986, vol. 46, 145
- **NER et al.** *DNA,* 1988, vol. 7, 127
- Remington 's Pharmaceutical Sciences. Mack Publishing Co, 1990
- **KAWADA et al.** Insights from advances in research of chemically induced experimental models of human inflammatory bowel disease. *World J. Gastroenterol.,* 2007, vol. 13 (42), 5581-5593
- **WIRTZ ; NEURATH.** Mouse models of inflammatory bowel disease. *Advanced Drug Delivery Reviews,* 2007, vol. 59 (11), 1073-1083
- **BONOIOTTO M. ; WJ JORDAN ; J. ESKDALE ; A. TOSSI ; N. ANTCHEVA ; S. CROVELLA ; ND CONNELL ; G GALLAGHER.** Human β-Defensin 2 Induces a Vigorous Cytokine Response in Peripheral Blood Mononuclear Cells. *Antimicrobial Agents and Chemotherapy,* 2006, vol. 50, 1433-1441
- **BOWDISH et al.** Immunomodulatory properties of defensins and cathelicidins. *Curr. Top. Microbiol. Immunol.,* 2006, vol. 306, 27-66
- **GERSEMANN et al.** Crohn's disease--defect in innate defence. *World J. Gastroenterol,* 2008, vol. 14, 5499-5503
- **LEHRER R.I.** Primate defensins. *Nat. Rev. Microbiol.,* 2004, vol. 2, 727-738
- **SWIDSINSKI et al.** Mucosal flora in inflammatory bowel disease. *Gastroenterology,* 2002, vol. 122, 44-54
- **NIYONSABA F. ; H. USHIO ; N. NAKANO ; W. NG ; K. SAYAMA ; K. HASHIMOTO ; I. NAGAOKA ; K. OKUMURA ; H. OGAWA.** Antimicrobial peptides human β-defensins stimulate epidermal keratinocyte migration, proliferation and production of proinflammatory cytokines and chemokines. *Journal of Investigative Dermatology,* 2007, vol. 127, 594-604
- **ROWLAND TL ; SM MCHUGH ; J DEIGHTON ; RJ DEARMAN ; PW EWAN ; I KIMBER.** Differential regulation by thalidomide and dexamethasone of cytokine expression in human peripheral blood mononuclear cells. *Immunopharmacology,* 1998, vol. 40, 11-20
- **WANG et al.** Host-microbe interaction: mechanisms of defensin deficiency in Crohn's disease. *Expert. Rev. Anti. Infect. Ther.,* 2007, vol. 5, 1049-1057
- **WEHKAMP et al.** Reduced Paneth cell alpha-defensins in ileal Crohn's disease. *Proc. Natl. Acad. Sci. U. S. A,* 2005, vol. 102, 18129-18134